# EUROPEAN PATENT APPLICATION

(11) **EP 4 790 138 A1**
(43) Date of publication of application: **12.08.2026**
(21) Application number: 25156168.4
(22) Date of filing: 06.02.2025
(51) Int. Cl.: C12N 11/02, C12N 11/12, C12N 11/14, C12N 9/20, C12N 9/34, C12P 7/20, C12P 7/52, C12P 7/625, C12P 7/6458, C12P 19/02, C12P 19/14

(54) **METHOD FOR IMMOBILIZATION OF AGENTS ON RICE HUSKS PARTICLES, RICE HUSKS PARTICLES WITH IMMOBILIZED AGENTS AND THEIR APPLICATION FOR USE IN CATALYZED REACTIONS**

(71) Applicant: Universita Degli Studi di Trieste, 34127 Trieste (IT); ViaZym B.V., 2629JD Delft (NL)
(72) Inventor: GARDOSSI, Lucia, 34127 Trieste (IT); DANIELLI, Chiara, 34127 Trieste (IT); VAN LANGEN, Luuk, 2629 JD Delft (NL)
(74) Representative: D'Agostini, Giovanni

(57) **Abstract**

Method for immobilization of one or more agents on a carrier comprising rice husks in which the method comprises the provision of rice husks particles, one or more agents to be immobilized on rice husks, preparation of a liquid solution in water of the one or more agents, wetting the rice husks particles with the liquid solution, mixing the mixture of agents' liquid solution and rice husks, drying, the rice husks being not subjected to chemical modifications or pre-treated with crosslinking additives, in which wet rice husk particles are obtained in which water molecules of the agents' liquid solution are adsorbed by the rice husk particles surface into cavities, tunnels and pores of the rice husk particles; use of the obtained rice husk particles with immobilized agents for reactions catalyzed by the immobilized agents; rice husk with one or more immobilized agent in the form of distinct fragments of rice husk containing the one or more agents in which the distinct fragments of rice husk have a size in the range 100-1000 micron and in that the distinct fragments of rice husk are free from chemical or crosslinking additives.

## Description

The present invention relates to a method for immobilization of enzymes on rice husk according to the characteristics of claim 1.

The present invention also relates to the use of rice husks particles with immobilized agents in catalyzed reactions according to the characteristics of claim 12.

The present invention also relates rice husks particles with immobilized agents according to the characteristics of claim 13.

### Prior art

Enzymes accept a wide variety of complex molecules, including synthetic molecules with structures very different from the substrates found in nature. The practical utilization of enzymes as biological catalysts (biocatalysts) is driven by their versatility, regio-, chemo-, and enantio-selectivity, along with the need for the chemical industry to move towards environmentally compatible catalysts and processes. An immobilized insoluble enzymatic formulation not only enables the reusability of the formulation, thus lowering production costs, but also reduces waste stream generation. There are several techniques for immobilizing enzymes on solid supports. The classical methods of immobilization on solid supports envisage contact between a solid support and an aqueous solution containing the dissolved enzyme. Suitable incubation between enzyme and solid support is followed by recovery of the immobilized enzyme on the solid support, for example by filtration. In the case when the immobilized enzyme is to be used in organic systems at low water activity, i.e. in the absence of free water molecules that could interfere with the synthetic reactions, it is necessary to remove the water in excess, for example by washing with acetone or evaporating the water under reduced pressure. Enzymes are always retaining some water strongly bound to the protein, to maintain their active conformation.

The attachment of the enzymatic protein on the support can take place either by establishment of weak interactions (ionic or van der Waals forces) or by formation of covalent bonds between functional groups present on the surface of the enzymatic protein, as for example amino groups of lysine residues, and appropriate reactive groups of the solid support, as for example epoxide groups. In the first case, that is establishing weak interactions, the enzymatic protein can be detached from the support in the presence of an aqueous or hydrophilic phase in which it will tend to be distributed or the enzymatic protein can be detached from the support on account of mechanical stresses (e.g. viscosity of the reaction mixture). To overcome this problem, methods have also been developed that envisage crosslinking between the enzyme particles adsorbed on the support or the formation of a coating of silicone intended to hold the enzyme on the solid surface.

Patent US 5,776,741 describes a method of immobilization of an enzyme that envisages the formation of granules containing the enzyme, which is maintained around a silica support through the use of a binder, such as polyvinylpyrrolidone (PVP) or gelatin. The immobilization method does not, however, envisage the formation of covalent bonds, therefore the preparation is recommended for applications in the presence of at least 50% of organic phase, in order to avoid damaging the enzymatic formulation with distribution of the enzymatic protein in the aqueous phase.

Scientific advances in biocatalysis have been boosted primarily by requests coming from the pharmaceutical industry and the fine chemistry sector, which make use of processes often characterized by low atom efficiency and high production of waste. Enzymes used for biocatalytic applications represent a limited share of the global enzyme market. Some studies, which have tried to analyze the enzyme market by segments indicate that industrial enzymes accounted for 56.9% of the global enzyme market in 2015. The term "industrial enzymes" refers to a wide range of applications in sectors including food and beverage, detergents, animal feed, textile, pulp and paper, nutraceuticals, personal care and cosmetics, and wastewater treatments, thus excluding biocatalysis, which is rather included in the category of "specialty enzymes". This latter segment embraces applications such pharmaceuticals, diagnostics, biocatalysis and biotechnological research, addressing lower volume and higher value products, with pharmaceutical applications being the dominant ones. Biocatalysis already contribute within the biorefinery context by transforming different conventional biomass and renewable feedstock into chemicals. Transformations of oils/fats, polysaccharides, lignocellulosic bio-mass and proteins are made possible by a wide array of enzymes. Research efforts are currently directed not only towards the transformation of biomass through more efficient routes, but also at the identification of new and nonconventional feedstock streams as opportunities. The food industry is a major driver of biocatalysis growth: lipases are used traditionally for transforming oils and fats in food ingredients such as cocoa butter analogues, and nowadays are profitably applied in the synthesis of valuable chemicals, including lubricants, esters for the cosmetic sector and surfactants. The food industry also makes use of proteases for converting peptides and proteins into shorter peptides used as supplements, ingredients of infant formulas or as pharmacologically active agents. On the other hand, optically pure amino acids obtained through enzymatic enantio-selection are massively used by the fine chemical industry as a chiral-pool, since the stereoselectivity of different enzyme classes allows for the bioconversion of cheap amino acids into chiral building blocks for the manufacture of expensive and chemically complex chiral drugs or fine chemicals. Biotransformation of sugars represents probably the oldest example of a biorefinery and, also in this case, the first technologies were developed for the food and beverage sector.

The amylase-catalyzed hydrolysis of starch dates back to the 1970s and was followed by glucose isomerization to fructose. Currently, they represent the largest enzymatic processes implemented at an industrial scale. The starch industry has capitalized on the know-how generated for polysaccharide processing and later promoted the surge of the first-generation biofuel industry. Only in the last two decades have scientific advances enabled the design, production and optimization of a pool of hydrolytic enzymes able to split the chemically heterogeneous glycosidic bonds of cellulose and hemicellulose. Conversely, starch based biorefineries are being replaced by second-generation biorefineries fed by non-food-based lignocellulosic feedstock. Technologies are known which allow conversion of non-food lignocellulosic feedstock into fermentable C5 and C6 sugars.

WO 2012 085206 discloses a method for covalent immobilization of enzymes on functionalized solid polymeric supports. This patent application reports the immobilization of lipases. In the first case the immobilization is stable due to covalent immobilization and the carrier is a commercial fossil based organic resin.

More recently, liquid formulations of a modified lipase from Thermomyces lanuginosus (TLL) were developed for the economically attractive conversion of waste oils into biodiesel. An engineered lipase TLL-T3 variant with three residue substitutions was developed to improve its acid tolerance for biodiesel production from acidified oil.

US 5,776,741 describes the immobilization method most commonly used by industry for lipase from Thermomyces Lanuginosus (TLL). It has been specifically developed for being applied in the process of interesterification of triglycerides. This formulation is made by silica particles agglomerated into granules in the presence of water-soluble binders (PVP, cellulose derivatives). Currently this formulation is the most widely employed by food industry for the production of cocoa butter analogues, spreading creams, margarine.

### Problems of the prior art

Prior art methods for carriers for industrial enzyme immobilization are subject to several problems and drawbacks.

The stable immobilization of enzymatic proteins is required in industrial processes where there is the risk of the enzymatic protein detachment because of the exposure to water, viscous systems, mechanical stress. Prior art methods for carriers for industrial enzyme immobilization require one or more of the following steps: chemical functionalization of the immobilization carrier to introduce chemical group able to form covalent bond with the protein surface / use of di-functional crosslinking agents, as for example glutaraldehyde, able to create links between the enzyme or protein particles after their adsorption on the carrier surface, thus preventing the detachment of the enzyme upon exposure to water or other stressing conditions / chemical functionalization of the carrier to introduce chemical groups able to establish metal affinity interactions with proteins expressed with histidine tags. These steps inevitably have an impact on the final result when the carrier and corresponding protein or enzyme are used as biocatalyst in corresponding processes so that the prior art method are not appropriate for industrial applications requesting low-toxicity.

In prior art methods which are based on the covalent modification of the enzyme for its stable binding on the carrier, such modifications cause a severe loss of enzymatic activity. Considering the method of US 5,776,741, a disadvantage is present, that is the disaggregation of the granules in the presence of traces of water. Moreover, the production of the formulation requires specific equipment, spay dryer or granulators, and the addition of the binders. Despite the wide number of studies present in the literature, very few examples of covalent bonding of lipases on solid supports have been reported so far because of their structural and conformational features. In general, only few protocols for the covalent immobilization of enzymes report satisfactory and clearly stated immobilization yields calculated on the basis of the enzymatic activity recovered after the immobilization process.

Crosslinking of enzymes, or enzymes that are previously adsorbed on the carrier surface are subject to problems of toxicity and regulatory limitations for food applications. The most widely employed crosslinking agent for protein is glutaraldehyde. For example, Lipase B from Candida antarctica (CaLB) can be immobilized covalently on chemically oxidized rice husks (RH) after the introduction of a hexamethylenediamine spacer (HMDA) and employing glutaraldehyde as bi-functional agent able to form imine bonds with the spacer and the amine groups of lysine residues present on the surface of the lipase. The toxicity of glutaraldehyde is widely documented although it is still used in food industry according to strictly defined official regulations. The adverse health effects on humans include sensitization of skin and respiratory organs and closed-systems are recommended to prevent hazards from glutaraldehyde exposure in industrial plants. The high toxicity of glutaraldehyde and its environmentally hazardous nature is anticipated to act as a major restraint for the market growth, since the use of this chemical is highly regulated by the government in respective regions, owing to health risks associated with it. It has been classified as a candidate substance of very high concern (SVHC) by European Chemicals Agency. Therefore, market players are focusing to lower dependency on glutaraldehyde.

Considering the method disclosed in WO 2012 085206, the use of commercial fossil based organic resin as a carrier, is expensive and not sustainable. Ready to use carriers, such as fossil-based resins and more specifically methacrylic resins bearing functional groups that allow for covalent binding of the enzymatic proteins are subject to both problems of environmental impact and costs. With reference to environmental impact, for example, it has been pointed out that the production of epoxy activated methacrylic resins represents the primary greenhouse gas emission source for the immobilized enzymes (51-83%), primarily because of the fossil based raw materials, namely glycidyl methacrylate and ethylene dimethyl acrylate. Both organic resins are commercially available: non-functionalized and activated, e.g. with epoxy or amino groups. The first type is much more expensive because of the complexity of their synthesis. They are also less sustainable because of the need of functionalizing agents (introduction of epoxy or other functional groups) with increasing of costs and also with effects on greenhouse gas emission. Less expensive commercial organic carriers, as e.g. polystyrene or polyphenolic resins, have no chemical functionalities on their surface, but they simply interact with the protein by weak non-covalent interactions leading to a non-firm attachment of the enzyme on the carrier, thus decreasing the stability of the enzymatic preparation. Finally, the stability of organic fossil-based carriers was reported to be unsatisfactory under chemical and mechanical stress.

Considering lipase from Thermomyces lanuginosus (TLL), one of the most widely employed formulation is the product named Lipozyme^{®} TL IM, which consists of agglomerates having a size of about 600 micron, made by the enzyme adsorbed on silica particles having a size < 100 micron with the aid of a binder. Granulation technology was used to immobilize lipase on silica granules, by first adsorbing the lipase on silica powder followed by agglomeration. Owing to the composition of the granulates, they are intended for use only in organic media with a water content < 50%. In an aqueous medium the lipase is desorbed and the particle slowly disintegrates, as stated by the producer. Therefore, they are mechanically stable for both batch and fixed bed column operation but only in low water media.

### Aim of the invention

The aim of the present invention is to provide renewable, sustainable, and less expensive carriers for industrial enzyme immobilization and a method for the effective and firm immobilization of agents, like for example enzymes, on these carriers.

### Concept of the invention

The aim is achieved by the characteristics of the main claim relative to the method, main claim relative to the rice husks particles, main claim relative to the use of the rice husks particles. The sub-claims represent advantageous solutions.

### Advantageous effects of the invention

The solution according to the present invention, presents various advantages.

The immobilization method according to the present invention allows agents, as for example, proteins or enzymes, to firmly bind on the carrier in the form of rice husk, preferably milled rice husk, without the need of physical-chemical modification or functionalization of the carrier or without the need of using binders or crosslinking agents, like toxic glutaraldehyde. The immobilization method according to the present invention allows to obtain a low toxicity when the carrier and corresponding protein or enzyme are used as biocatalyst in corresponding processes so that the resulting carrier and corresponding protein or enzyme appear particularly appropriate for industrial applications requesting low toxicity.

The immobilization method according to the present invention is less expensive if compared with prior art methods, it is easily scalable and no special equipment is required. Advantageously, the immobilization method according to the present invention is less expensive because rice husk has an extremely low cost and its accumulation or incineration represent a major environmental problem.

The immobilization method according to the present invention allows the enzymes immobilized on the corresponding carrier to retain their activity.

The present method provides a recyclable carrier with firmly immobilized enzymes. This is particularly relevant for the immobilization of glucoamylase because it is used in aqueous solution and no firmly immobilized and cost effective glucoamylase exists on the market. Similarly, currently there is no immobilized lipase on the market which provides evidence of the absence of protein leaching upon application in aqueous media.

Different lipases covalently immobilized on Immobeads are commercialized with name of Immozymes^{™} by ChiralVision^{™}. For instance, the commercial product Lipase 76546 commercialized by Sigma^{™}, is made by lipase TLL immobilized on the polyacrylic epoxy carrier Immobead 150. However only the specific activity is provided in the technical sheet. Advantageously, after use, the biocatalyst, composed by the agents and rice husk can undergo bio-composting to produce biogas and compost, provided that no toxic reagents have been employed in the production process using the carrier with firmly immobilized biocatalyst. This is a great advantage with respect to traditional carriers made by fossil-based resins because the inventive method provides the biocompostability not only of the enzyme but also of the carrier.

The immobilization method according to the present invention allows the use of a largely available renewable biomass material usable without any significant pre-treatment or modification. That leads to many advantages as extremely low costs, possibility to replace fossil based materials and chemicals, reduction of water, solvents, energy use in immobilization procedures, easier disposal of the carrier at its end-of-life, being natural, biodegradable, and with no toxicity.

With reference to the previously cited Lipozyme^{®} TL IM, the invention leads to the following advantages: the immobilized enzyme does not detach from the carrier upon exposure to water media, no need of binders, diluted enzymatic solutions, special equipment for the granulation and drying of the immobilized biocatalyst, mechanical, chemical and physical robustness of the immobilized enzyme that is distributed on particles that do not disaggregate.

Rice husks (RH) particles used as a carrier in the present invention confer to the biocatalyst the additional advantage of being made by a single robust fragment of composite material, which does not undergo disaggregation or the formation of fines under physical stress. Advantageously, with reference to the end of the use cycle of rice husks, they are easily biodegradable in soil and recyclable and in the literature different uses of rice husks are reported in building materials or for biogas production.

### Definitions

In the present description and in the appended claims the following terms must be understood according to the definitions given below.

The term "TLL" must be understood as referring to lipase from Thermomyces lanuginosus. The term "CaLB" must be understood as referring to lipase B from Candida antarctica.

The term "carrier" must be understood as referring to machined rice husks used for the immobilization process according to the present invention.

The term "agent" must be understood as comprising any agent able to be immobilized on rice husks for successive use of the product thus obtained in subsequent processes. Exemplary agents for the use in the method of immobilization according to the invention are enzymes, as glucoamylase from Aspergillus niger, lipase from Thermomyces lanuginosus (TLL), lipase B from Candida antarctica (CaLB), natural enzymes extracted or present in living organism, enzymes obtained by genetic engineering and manipulation, enzymes belonging to any class of the six classes defined by the International Union of Biochemistry (IUBMB): EC 1 Oxidoreductases, EC 2 Transferases, EC 3 Hydrolases, EC 4 Lyases, EC 5 Isomerases, EC 6 Ligases / synthetases, any protein or biological macromolecule able to catalyze a chemical reaction, bio-stimulants for agriculture, agent for bioaugmentation for soil for use in agriculture, nutrients as for example ammonia, phosphate, biofertilizers and biopesticides for the soil or plants for use in agriculture, microbiota for soil improvement.

The term "residual water" must be intend not as indicating a visible liquid phase residual water but as indicating residual water in the form of humidity, namely hydration water consisting in water molecules strongly interacting with the rice husk particles through hydrogen bonds, as for example bound on cellulose or silica of the rice husk particles and bound on the agents. When referring to rice husk particles which have not been subjected to chemical modifications and rice husk particles which have not being pre-treated with crosslinking additives, it will be understood by experts in the sector that rice husk particles do not comprise epoxide groups, binder, such as polyvinylpyrrolidone (PVP), cellulose derivatives, hexamethylenediamine (HMDA), glutaraldehyde and rice husk particles are not chemical functionalized to introduce chemical groups able to form covalent bond with agents, as for example chemically oxidized rice husks (RH).

The terms "enzyme" and "enzymatic protein" are to be considered as synonyms. In a physical sense, the term "binding" is referred to taking two things and tying them together, but in such a way that tie can be broken. In a physical sense, the term "bonding" is referred to joining two things and unifying them, making them much harder to separate.

### Description of the drawings

In the following a method is described with reference to the enclosed drawings, which are to be considered as a non-exhaustive example of the present invention in which:
Fig. 1 is a block diagram illustrating the method for firmly immobilization of enzymes on rice husk according to the present invention.
Fig. 2 schematically represents rice husk with particle size 200-400 micron before the application of the method for firmly immobilization of enzymes on rice husk according to the present invention.
Fig. 3 schematically represents an optical microscopy image of rice husk of Fig. 2.
Fig. 4 schematically represents dry rice husk with particle size 200-400 micron after the application of the method for firmly immobilization of enzymes on rice husk according to the present invention, as for example 1.
Fig. 5 schematically represents an optical microscopy image of rice husk of Fig. 4.
Fig. 6 illustrates the results of an application of the enzymatic preparation immobilized according to example 1 for the hydrolysis of maltose to glucose in aqueous medium under continuous flow conditions.
Fig. 7 illustrates the results of an application of the enzymatic preparation immobilized according to example 1 for the hydrolysis of maltose to glucose in aqueous medium in batch system.
Fig. 8 illustrates the enzymatic interesterification of triolein and tristearin.
Fig. 9 illustrates DSC thermograms of the raw products of the interesterification between triolein and tristearin for a sequence of synthetic cycles.
Fig. 10 illustrates a 1H-NMR spectrum of a sample obtained from the interesterification.
Fig. 11 illustrates the detected hydrolytic activity according to example 9 for the raw reaction mixture recovered after each synthetic cycle of the enzymatic interesterification and after removal of the immobilized enzyme.
Fig. 12 illustrates a DSC Analysis of the interesterification of tristearin and triolein with TLL immobilized on rice husk.
Fig. 13 illustrates a 1H-NMR spectrum of a sample obtained from interesterification.
Fig. 14 illustrates a 1H-NMR spectrum in CDCl3 of raw reaction product from example 8.
Fig. 15 illustrates a ESI-MS positive ion mass spectrum of the raw reaction product relative to the polycondensation products of dimethyl itaconate (A) and butanediol (B) catalyzed by lipase B from Candida antarctica (CaLB) immobilized according to example 6.
Fig. 16 is a table illustrating the activity results relative to example 4B of application of the enzymatic preparation immobilized on rice husks according to example 2 for the hydrolysis of maltose to glucose in aqueous medium in a batch system.
Fig. 17 is a table showing Lipase TLL released during the 8 interesterification cycles, assessed by measuring the hydrolytic activity present in the raw reaction product obtained from the interesterification reaction (EXAMPLE 9) of triolein and tristearin using TLL immobilized as described in EXAMPLE 5B.
Fig. 18A and Fig. 18B represent microscope images of the TLL immobilized on rice husk respectively before and after the interesterification process.
Fig. 19A illustrates DSC thermograms of the raw products of the interesterification between triolein and tristearin for a sequence of synthetic cycles performed using lipase TLL immobilized on rice husks in the presence of polyvinylpyrrolidone (PVP-TTL), according to prior art methods.
Fig. 19B illustrates DSC thermograms of the raw products of the interesterification between triolein and tristearin for a sequence of synthetic cycles performed using lipase TLL immobilized on rice husks in the presence of hydroxyethyl cellulose (HEC-TTL). according to prior art methods.
Fig. 20 is a table showing the Enzymatic U detected after each hydrolytic cycle as a result of the detachment of the protein from the carrier with reference to example 9, that is the assessment of the leaching of the active enzyme from the support when the immobilized lipases TLL (A) and CALB (B) immobilized on rice husk according to examples 5 and 6 are exposed to an aqueous emulsion. Data compare lipases immobilized on rice husk with the same lipases covalently immobilized on commercial epoxy acrylic resins.
Fig. 21A illustrates hydrolytic activity of TLL immobilized on rice husk according EXAMPLE 5B assessed for a 8-months period washed before subsequent drying and storage.
Fig. 21B illustrates hydrolytic activity of TLL immobilized on rice husk according EXAMPLE 5B assessed for a 8-months period without washing before subsequent drying and storage.
Fig. 22A illustrates DSC thermograms of the raw products of the interesterification, as for example 7, for a sequence of 8 consecutive synthetic cycles relative to a process catalyzed by TLL immobilized on rice husk according to EXAMPLE 5B and washed with water after drying and prior to final drying and storage.
Fig. 22B illustrates DSC thermograms of the raw products of the interesterification, , as for example 7, for a sequence of 8 consecutive synthetic cycles relative to a process catalyzed by the same immobilized enzyme but stored without being washed with water.
Figure 23 represents Hydrolytic activity (tributyrin hydrolysis) of lipase TLL immobilized on rice husk according EXAMPLE 5B, measured before the starting of the interesterification process and after 8 cycles of 24h at 70°C. The two samples of TLL where prepared according EXAMPLE 5B but only one (left column in the figure) was subjected to the rinsing procedure with water after immobilization and drying, and priori to final drying and storage.
Fig. 24 is a table showing the results of subsequent cycles in which the % of recovered enzymatic activities is reported with reference to example 9, that is the assessment of Hydrolytic activity of TLL (A) and CALB (B) immobilized on rice husk used in aqueous emulsion for the hydrolysis of tributyrin and recovered activity upon recycling. Data compare lipases immobilized on rice husk with the same lipases covalently immobilized on commercial epoxy acrylic resins.

### Description of the invention

In general, the present invention is relative to a method for immobilization of one or more agents, as for example enzymes or proteins or biocatalysts or supplements or other additives, on rice husks or rice husks particles and their application for use of the agents in subsequent processes. The immobilization method and corresponding conditions allow a certain amount of one or more agents, as for example enzymes particles, to firmly bind on the carrier, without the need of previous physical-chemical modification or functionalization of the solid material, i.e. the carrier, or without the need of the using binders or crosslinking agents, as for example toxic glutaraldehyde. Because of the low toxicity of the materials and the possibility of reusing the rice husk at the end of the biocatalyst life cycle, this method appears particularly appropriate for industrial applications requesting low-toxic, inexpensive and sustainable solutions. The method for immobilization of agents on rice husk particles provides the agent to be solubilized in water and distributed on the solid carrier by gentle mixing to form wet hydrated particles where the agents' solution has been adsorbed on the rice husk surface or penetrated and absorbed into the cavities, tunnels and pores of the carrier material. Rice husks particles are finally dried slowly and under mild conditions to maintain only water molecules that hydrates the material and the agent so that no bulk water is present. No special equipment is required for the implementation of the method. During these steps the agent firstly adsorbs onto the rice husk surface and penetrates absorbing into cavities, tunnels and pores of the carrier material, that is rice husk and then establishes strong interactions between the lignocellulosic surface and the silica of the matrix of the rice husks particles and the agent. The presence of silica is specific of rice husk, as compared to others lignocellulosic biomasses. We believe it plays a role in the firm immobilization of the protein. The method implies the loading of a low amount of agents, like for example enzyme or protein, per gram of carrier but spread on a large surface, being the carrier rich of cavities and silica, and with a density lower than 0.4 g/mL. The product obtained from the method of the present invention retains activity when exposed to low-water or hydrophobic media and also when exposed to aqueous media and physical stress. Following test, formulations showed good retained activity properties when exposed to aqueous media having a pH in the range 4.5 to 8.0. Applications of enzymes in an aqueous environment require a firm immobilization of the enzyme on the carriers for preventing the detachment of the protein and its partition in the medium.

Rice husk is an extremely robust composite material made by about 25-35% w/w of cellulose, about 15 -17% w/w of silica, about 18-21% w/w of hemicellulose, and about 26-31% w/w of lignin. Because of the high chemical and mechanical stability of rice husk, conferred by silica and lignin, rice husk with firmly immobilized enzymes according to the invention can be used for applications in various reactions media, as for example aqueous reaction media, organic reaction media, using different reaction configurations, as for example batch, continuous flow, thin-film, viscous solvent-less systems, bubble aeration in a rotating bed reactors, turbo-reactors and thin-film processes, column reactors, any other reactor used in industry, and different substrates. Rice husk meets circularity criteria because it can be reutilized at the end of its industrial application. Finally, as natural material, it has the subsidiary advantage that it is subjected to less stringent legislative constraints even after chemical modification. The tubular structure of tracheids confers to rice husks a very low density, lower than 0.4 g/mL, while silica is responsible for its remarkable mechanical robustness.

The method according to the present invention represents the first example of immobilization procedure that allows to firmly immobilize a certain amount of protein molecules on a solid matrix without the need of using one of the following procedures, singularly or in combination: i) chemical functionalization of the immobilization carrier to introduce chemical group able to form covalent bond or ionic bonds with the protein surface; ii) use of di-functional crosslinking agents (e.g. glutaraldehyde) able to create links between the protein particles as they are or after their adsorption on the carrier surface, thus preventing the detachment of the enzyme upon exposure to water or other stressing conditions; iii) chemical functionalization of the carrier to introduce chemical groups able to establish metal affinity interactions with proteins expressed with histidine tags.

Advantageously, the method does not require to modify covalently the enzyme for its firm binding on the carrier. Such covalent modifications and the subsequent covalent bonding on the carrier may cause a severe loss of enzymatic activity. On the contrary, the enzymes immobilized according the present invention retain their activity, and this is evident in the immobilization of lipase from Thermomyces lanuginosus (TLL) where the lipase after immobilization expresses an even higher activity (111%) with respect to the native enzyme, as a consequence of the mild immobilization procedure and the preservation of the conformational freedom required by lipase from Thermomyces lanuginosus (TLL) to express its full activity.

The firm immobilization of enzymes is required in industrial processes where there is the risk of protein detachment because of the exposure to water, viscous systems, mechanical stress. The present method provides a stable immobilized biocatalyst, recyclable. This is particularly relevant for the immobilization of glucoamylase and lipases, because currently there are no formulations of such enzymes on the market that demonstrate the firm immobilization of the enzymes. Advantageously rice husk is available worldwide in large quantities, the inventive method of immobilization is easily scalable and no special equipment is required. The method is applicable to different industrial enzymes useful in industrially relevant processes. Scientific advances in biocatalysis have been boosted primarily by requests coming from the pharmaceutical industry and the fine chemistry sector. The term "industrial enzymes" refers to a wide range of applications in sectors including food and beverage, detergents, animal feed, textile, pulp and paper, nutraceuticals, personal care and cosmetics, and wastewater treatments, thus excluding biocatalysis, which is rather included in the category of "specialty enzymes". This latter segment embraces applications such pharmaceuticals, diagnostics, biocatalysis and biotechnological research, addressing lower volume and higher value products, with pharmaceutical applications being the dominant one. Biocatalysis already contribute within the biorefinery context by transforming different conventional biomass and renewable feedstock into chemicals. Transformations of oils/fats, polysaccharides, lignocellulosic bio mass and proteins are made possible by a wide array of enzymes. The food industry is a major driver of biocatalysis growth: lipases are used traditionally for transforming oils and fats in food ingredients. Enzymes are profitably applied in the synthesis of valuable chemicals, including lubricants, esters for the cosmetic sector and surfactants. On the other hand, optically pure amino acids obtained through enzymatic enantio-selection are massively used by the fine chemical industry as a chiral-pool, for bioconversion of cheap amino acids into chiral building blocks for manufacturing expensive and chemically complex chiral drugs or fine chemicals. Biotransformation of sugars represents the oldest example of a biorefinery and, also in this case, the first technologies were developed for the food and beverage sector. It is evident that there is a wide potential for biocatalytic proteins in fermentation worldwide and for expanding the benefits of biocatalysis to the emerging bio-based chemistry sector, thus boosting bioeconomy as a whole.

Advantageously the method according to the present invention does not provide the use of aldehyde groups usable for the direct covalent bonding of proteins or water-soluble binders.

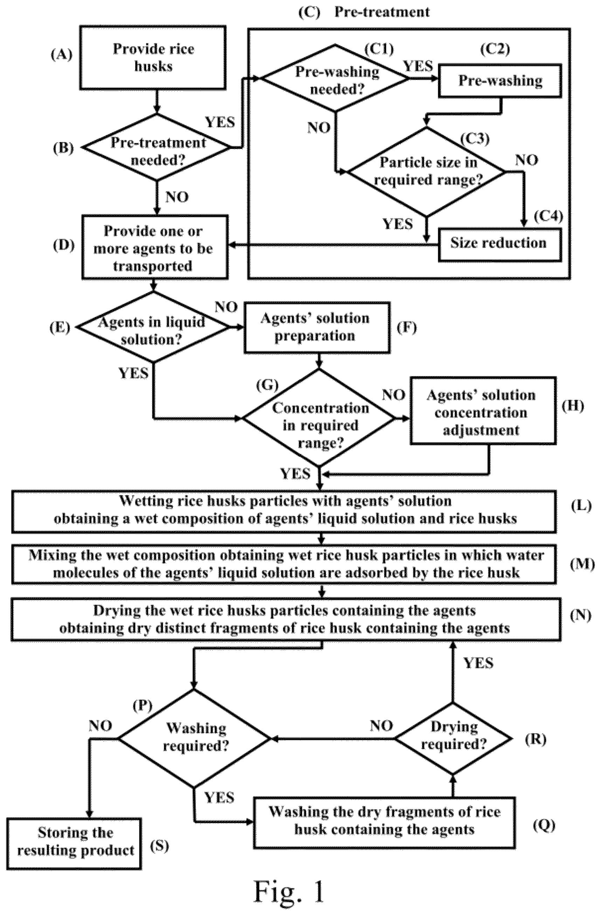

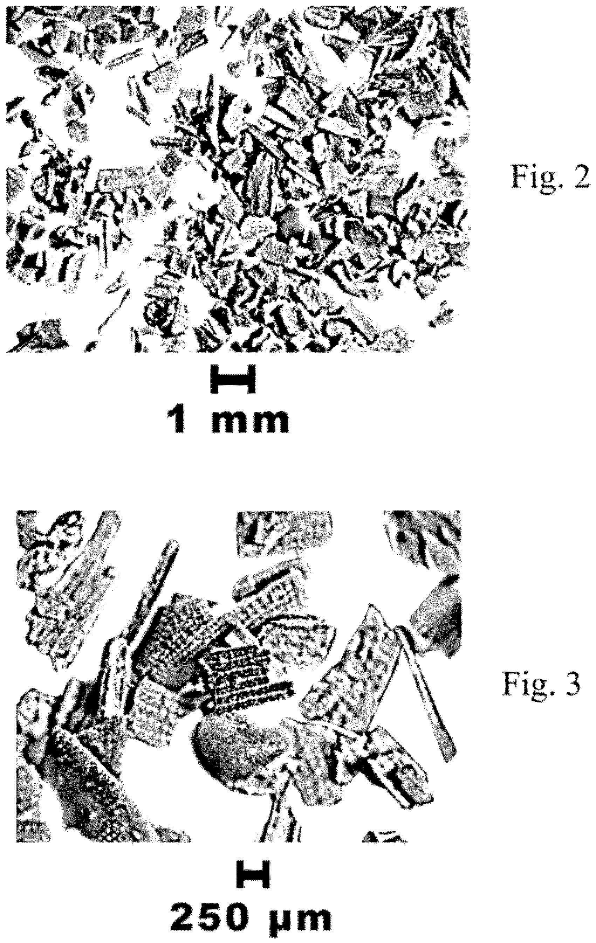

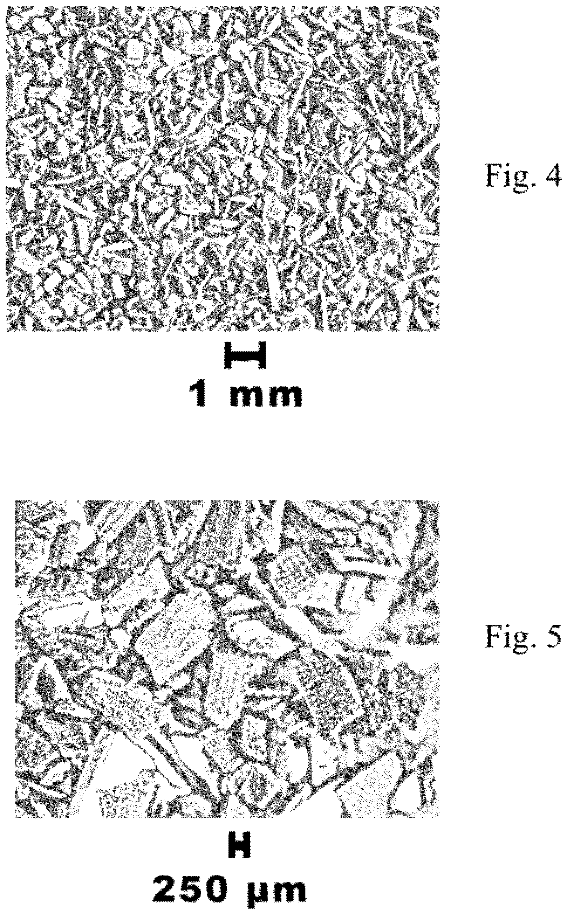

EXAMPLE 1: immobilization of glucoamylase from Aspergillus niger on milled rice husk, with a loading of 2 mg / g of dry of carrier. Enzyme of class glucoamylase from Aspergillus niger as a commercially available solution was provided to be used as agent to be transported by the rice husks. In detail a quantity of 0.1 mL of a commercially available solution containing glucoamylase with a protein content of 82 mg/mL was used. An example of a commercially available solution is the product named Dextrozyme^{®} GA from Novozymes^{®}. The commercially available solution containing glucoamylase was diluted with water to reach a protein concentration of 1.6 mg/mL, with obtainment of an agent solution in the form of an aqueous glucoamylase solution. The obtained concentration was checked with a Bradford test. The volume was selected in order to reach an optimal hydration of the support upon the immobilization of the enzyme on the rice husk, without forming a distinct aqueous liquid phase. Rice husks machined to obtain a particle size in the range 200-400 micron were provided (Fig. 1, Fig. 2). A quantity of 400 mg of rice husks was wetted with 500 micro-liters of the agent solution in the form of aqueous glucoamylase solution obtaining a wet composition of rice husks and agent solution of glucoamylase. The resulting wet composition was mechanically mixed. to form homogeneously hydrated rice husk particles, that is a mixture of wet rice husks particles with no distinct liquid phase visible and the water, once adsorbed onto the rice husk surface or absorbed penetrating into the cavities, tunnels and pores of the carrier material hydrates the material and the agent so that no bulk water is present. With reference to the term "bulk water" it must be underlined that, from a chemical point of view, the water can be classified by the separation from the protein surface into bulk water and hydration water. Hydration water interacts closely with the protein and contributes to protein folding, stability and dynamics, as well as interacting with the bulk water. Bulk water is intended as being water which is free to move. On the contrary, hydration water forms water networks around the protein surface to keep protein in solution. Individually bound water has multiple contacts that stabilize the protein structure. This makes the method different from known ones. The resulting mixture of wet rice husks particles was dried in order to gently remove only those water molecules which are not strongly bound on the support or the agent. Drying was performed by spreading the resulting rice husk fragments on a drying surface and performing a drying procedure on the air at a drying temperature for a certain drying time. In this example, drying temperature was 25°C and drying time was 24 hours. After the drying procedure distinct fragments of rice husk containing the enzyme were obtained, together with residual water molecules hydrating the support and the enzyme, corresponding to about 9% w/w with respect to the weight of the distinct fragments of rice husk containing the enzyme. The drying procedure is provided to last for at least a minimum drying time in order to allow the creation of interactions between the lignocellulosic matrix and the silica of rice husks and the enzyme. No aggregates were observed. Prior art methods for the drying of immobilized enzymes make use of high temperature or solvents can promote enzyme inactivation, besides interfering with hydrogen bonds created between the agent and the rice husk. After drying, the distinct fragments of rice husk containing the enzyme were subjected to a washing step in which the distinct fragments of rice husk containing the enzyme were resuspended in 5 mL of potassium phosphate buffer (0.1 M, pH 8) and incubated under orbital mixing for 10 minutes to remove the protein particles, that is the enzyme particles, not firmly attached on the material, that is on the distinct fragments of rice husk after the drying procedure. The supernatant is removed by decanting. The washing step can be repeated further times, as for example one further time or two or more further times, depending on the possible final application of the obtained product. The obtained product (Fig. 4, Fig. 5), comprising the distinct fragments of rice husk with the immobilized glucoamylase enzyme, was stored at 5°C suspended in a 0.05M buffer pH 7 (1 g in 5mL).

EXAMPLE 2: immobilization of glucoamylase from Aspergillus niger on milled rice husk, with a loading of 100 mg / g dry of carrier. Enzyme of class glucoamylase from Aspergillus niger as a commercially available solution was provided to be used as agent to be transported by the rice husks. In detail a quantity of 500 micro-liters of a commercially available solution containing glucoamylase with a protein content of 82 mg/mL was used without any dilution. An example of a commercially available solution is the product named Dextrozyme^{®} GA from Novozymes^{®}. In this case the 500 micro-liters of commercially available solution containing glucoamylase were not further diluted so that the agent solution in the form of an aqueous glucoamylase solution consists of the commercially available solution in the original concentration. Rice husks machined to obtain a particle size in the range 200-400 micron were provided. A quantity of 400 mg of rice husks was wetted with 500 micro-liters of the agent solution in the form of 500 micro-liters of commercially available solution containing glucoamylase obtaining a wet composition of rice husks and agent solution of glucoamylase. The resulting wet composition was mechanically mixed to form homogeneously hydrated rice husk particles, that is a mixture of wet rice husks particles with no distinct liquid phase visible and the water, once adsorbed onto the rice husk surface or absorbed penetrating into the cavities, tunnels and pores of the carrier material, hydrates the material and the agent so that no bulk water is present. This makes the method different from known ones. The resulting mixture of wet rice husks particles was dried in order to gently remove only those water molecules which are not strongly bound on the support or the agent. Drying was performed by spreading the resulting rice husk fragments on a drying surface and performing a drying procedure on the air at a drying temperature for a certain drying time. In this example, drying temperature was 25°C and drying time was 24 hours. The drying procedure is provided to last for at least a minimum drying time in order to allow the creation of interactions between the lignocellulosic matrix and the silica of rice husks and the enzyme. Prior art methods make use of high temperature or solvents can promote enzyme inactivation, besides interfering with hydrogen bonds created between the agent and the rice husk. After the drying procedure distinct fragments of rice husk containing the enzyme were obtained, together with residual water molecules hydrating the support and the enzyme, corresponding to about 9% w/w with respect to the weight of the distinct fragments of rice husk containing the enzyme. The drying procedure is provided to last for at least a minimum drying time in order to allow the creation of interactions between the lignocellulosic matrix and the silica of rice husks and the enzyme. No aggregates were observed. After drying, the distinct fragments of rice husk containing the enzyme were subjected to a washing step in which the distinct fragments of rice husk containing the enzyme were resuspended in 5 mL of potassium phosphate buffer (0.1 M, pH 8) and incubated under orbital mixing for 10 minutes to remove the protein particles, that is the enzyme particles, not firmly attached on the material, that is on the distinct fragments of rice husk. The supernatant is removed by decanting. The washing step can be avoided or repeated further times, as for example one further time or two or more further times, depending on the possible final application of the obtained product. The obtained product comprising the distinct fragments of rice husk with the immobilized glucoamylase enzyme, was stored at 5°C suspended in a 0.05M buffer pH 7 (1 g in 5mL).

GLUCOSE CONCENTRATION ANALYSIS PROCEDURE: the following procedure is used to analyze the glucose concentration of the samples resulting from reactions described in the examples 3 and 4. The analysis is made on a sample which is a reaction product resulting from a reaction providing the use of an obtained product from examples 1 or 2, consisting of distinct fragments of rice husk with the immobilized glucoamylase enzyme. For the glucose concentration analysis, a solution of 55 mg ABTS (2 mM) in 50 mL phosphate buffer, pH 7, 0.05 M is prepared. Then, to the solution are added 0.2 mL of a 1 mg/mL glucose oxidase solution in demineralized water, and 0.2 mL of a 1.5 mg/mL horse radish peroxidase solution in demineralized water. A quantity of 2.95 mL of this solution is introduced in a UV-Vis cuvette together with 0.05 mL of the sample which is a reaction product resulting from a reaction providing the use of an obtained product from examples 1 or 2. Then the absorbance at 405 nm is followed for 5 minutes. The variation in absorbance relative to the sample is compared with the variation in absorbance of a 5 mM glucose aqueous reference solution. The glucose concentration in each sample is calculated by the formula eq.1: $\left[glucose\right] \left(mM\right) = \frac{Δ {A}_{405 , sample} \left({min}^{- 1}\right)}{Δ {A}_{405 , ref} \left({min}^{- 1}\right)} \times \frac{{\left[glucose\right]}_{ref} \left(mM\right) \times {V}_{ref} \left(mL\right)}{{V}_{sample} \left(mL\right)} \times df$ In which:
- ΔA_{405,sample} is the variation in absorbance of the sample;
- ΔA_{405,ref} is the variation in absorbance of the reference solution;
- [glucose]_{ref} is the glucose concentration of the reference solution;
- V_{ref} is the volume of reference solution;
- Vₛₐₘₚₗₑ is the volume of diluted sample solution;
- df is the dilution factor of the sample solution.

The glucose concentration is then plotted in a [glucose] vs time graph. The activity of the enzyme preparation is calculated by the formula eq.2: $Activity \left(U {g}^{- 1}\right) = \frac{\frac{Δ \left[glucose\right] \left(mM {min}^{- 1}\right)}{2} \times {V}_{assay} \left(mL\right) \times 1000}{{g}_{sample}}$ In which:
- Δ[glucose] is the rate of glucose formation, which is the slope of the [glucose] vs time curve;
- V_{assay} is the volume of the maltose hydrolysis reaction;
- gₛₐₘₚₗₑ is the weight of immobilized enzyme used in the reaction.

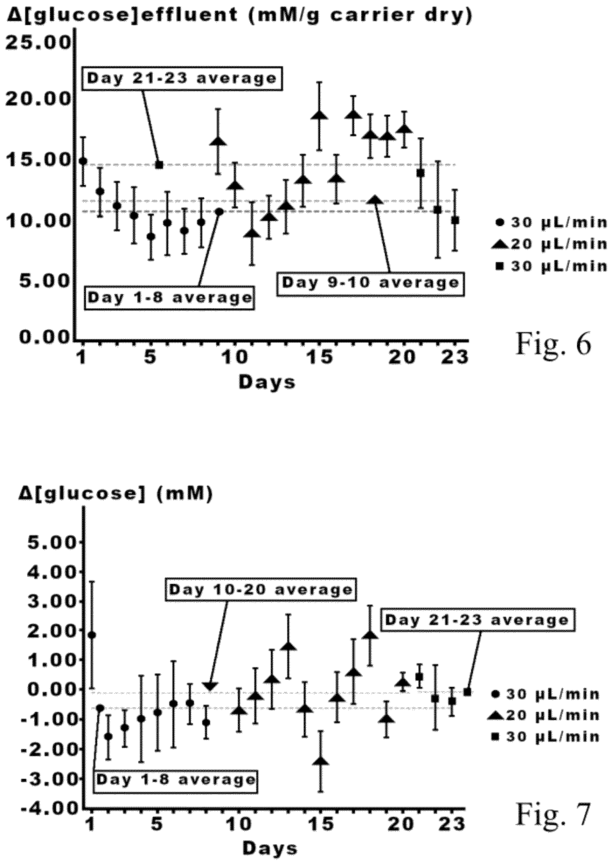

EXAMPLE 3: Application of the enzymatic preparation immobilized on rice husks according to example 1 for the hydrolysis of maltose to glucose in aqueous medium under continuous flow conditions. About 1.25 g of the suspended obtained product from example 1, containing the fragments of rice husk with the immobilized glucoamylase enzyme, were provided after storage. The indicated quantity of 1.25 g of the suspended obtained product from example 1 corresponds to 0.47 g of distinct fragments of rice husk with the immobilized glucoamylase enzyme and 0.68 U total. The indicated quantity of 1.25 g of the suspended obtained product from example 1 was introduced in a glass column having dimensions 10 cm x 1 cm. A 100 mL substrate solution was prepared by dissolving 26.3 g of maltose mono hydrate in 10 mM citrate buffer (pH 4.5). The solution was pumped through the column at a flow rate of 0.03 mL/min or at a flow rate of 0.02 mL/min for 23 days. The reaction was monitored at 25°C, collecting samples of the effluent every 24 hours. The effluent was analyzed to determine the glucose concentration in each sample, with the previously described Glucose Concentration Analysis Procedure and the results of the Glucose Concentration Analysis Procedure are plotted in Fig. 6, representing the measured produced glucose per g of dry enzyme preparation in the effluent of the continuous flow experiment of the non-crosslinked glucoamylase immobilized on rice husk. By decreasing the flow rate from 0.03 mL/min to 0.02 mL/min, the contact time between the substrate solution and the immobilized enzyme of the obtained product from example 1 increases, which leads, as expected, to an higher glucose concentration (average) measured in the effluent. Increasing the flow rate back to 0.03 mL/min causes a decrease in the measured glucose concentration, as expected. This confirms that the observed hydrolysis of maltose is catalyzed by the immobilized enzyme and the observed hydrolysis of maltose is not the result of the degradation of the substrate for the effect of temperature or other external factors. Notably, 100 mg of pristine carrier incubated in 5 mL of the substrate solution for 150 min at 25°C leads to no hydrolysis of maltose. The leaching of the enzyme from the solid support is determined by analyzing the glucose concentration in the same sample twice: at sample collection, and after incubating the sample at 25°C for 24 hours, and then subtracting the initial value from the final value. The resulting value are indicated ad Δ[glucose] and are plotted in Figure 7 representing the results of the leaching evaluation experiments for the continuous flow column. In total, the column was maintained in operation for 23 days, and no significant leaching was observed.

EXAMPLE 4A: Application of the enzymatic preparation immobilized on rice husks according to Examples 1 for the hydrolysis of maltose to glucose in aqueous medium in a batch system. A maltose solution in 10 mM citrate buffer pH 4.5 was prepared in the following way: 26.3 g of D-Maltose monohydrate and 0.19 g of citric acid were dissolved in 60 mL of demineralized water. The pH of the solution was adjusted to 4.5 using 1 M NaOH, and water was added to get a final volume of 100 mL. A quantity of 150 mg of wet immobilized glucoamylase immobilized on rice husk prepared and stored according to Example 1 was suspended in 5 mL of the maltose solution. The mixture is incubated and mixed for 3 hours in an orbital shaker. Samples of 100 micro-liters of the supernatant of the thus obtained mixture were taken every 30 minutes and diluted 2 times with 0.2 M HCl. The diluted samples were analyzed for glucose concentration with the previously described Glucose Concentration Analysis Procedure. The activity of the immobilized glucoamylase prepared according to Example 1 was 1.44 ± 0.22 U per g of dry carrier, corresponding to an immobilization yield of 21%, calculated as the ratio between the enzymatic U expressed by the immobilized glucoamylase and the enzymatic U loaded on the carrier. The pristine carrier, that is rice husk without any enzyme and not subjected to the immobilization process according to the present invention, was also tested and it expressed no activity in experiments carried out under the same conditions.

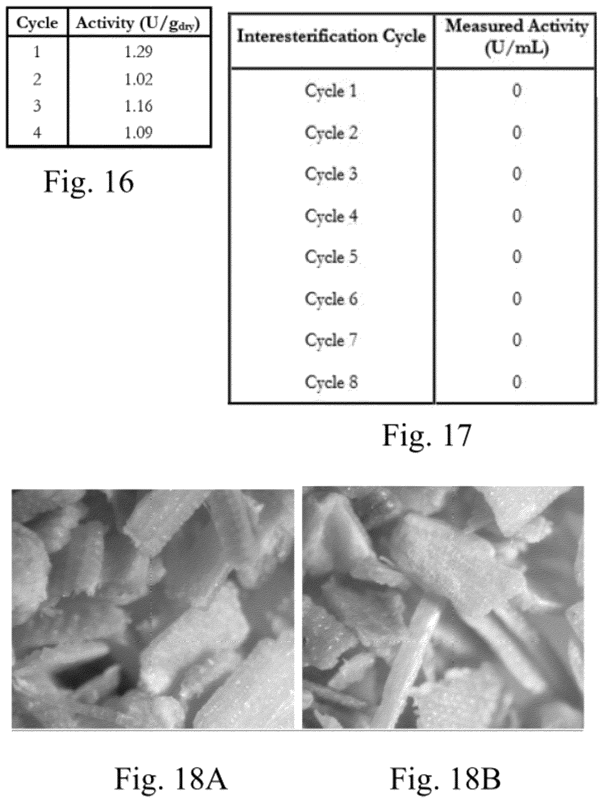

EXAMPLE 4B: Application of the enzymatic preparation immobilized on rice husks according to Example 2 for the hydrolysis of maltose to glucose in aqueous medium in a batch system. A maltose solution in 10 mM citrate buffer pH 4.5 was prepared in the following way: 26.3 g of D-Maltose monohydrate and 0.19 g of citric acid were dissolved in 60 mL of demineralized water. The pH of the solution was adjusted to 4.5 using 1 M NaOH, and water was added to get a final volume of 100 mL. A quantity of 150 mg of wet immobilized glucoamylase immobilized on rice husks prepared and stored according to Example 2 were suspended in 5 mL of the maltose solution. The mixture is incubated and mixed for 3 hours in an orbital shaker. Samples of 100 micro-liters of the supernatant of the thus obtained mixture were taken every 30 minutes and diluted 2 times with 0.2 M HCl. The diluted samples were analyzed for glucose concentration with the previously described Glucose Concentration Analysis Procedure. The activity of the immobilized glucoamylase prepared according to Example 2 was 1.29 ± 0.02 U per g of dry carrier. The immobilized enzyme was tested for four consecutive cycles, and after each cycle the immobilized biocatalyst was re-suspended in demineralized water (35 mL per g of biocatalyst) and incubated for 10 minutes at 25°C under orbital shaking. The activity results are presented in the table of Fig. 16, representing the results of the batch activity assay of glucoamylase immobilized according to Example 2, employed in the hydrolysis of maltose. The pristine carrier, that is rice husk without any enzyme and not subjected to the immobilization process of the invention, was also tested and it expressed no activity in experiments carried out under the same conditions.

HYDROLYTIC ACTIVITY EVALUATION PROCEDURE: the following procedure is used to analyze the hydrolytic activity of the samples resulting from reactions described in the examples 6 and 7. First a test aqueous environment is prepared composed by an Arabic gum-based emulsifier solution, tributyrin and a buffer, a KPi buffer which is a buffer made up of two potassium phosphate salts, monopotassium phosphate and dipotassium phosphate, in water. The assay is a standard procedure using KPi 0.1 M, pH 7.0. The Arabic gum-based emulsifier solution is prepared dissolving 0.6 g of Arabic gum in 6 mL of purified deionized water, as for example Milli-Q^{®} water, obtaining a solution of Arabic gum; then, 1.79 g of NaCl and 0.041 g of KH₂PO₄ are dissolved in 30 mL of purified deionized water, as for example Milli-Q^{®} water, obtaining a salt solution; then the solution of Arabic gum and 54 mL of glycerol are added to the salt solution; the pH is adjusted to 4.5 ± 0.05 using NaOH or HCl 1 M and then the volume is adjusted to 100 mL using purified deionized water, as for example Milli-Q^{®} water, thus obtaining the Arabic gum-based emulsifier solution. A tributyrin emulsion is prepared by first mixing together 5 mL of tributyrin, 17 mL of Arabic gum-based emulsifier solution and 78 mL of purified deionized water, as for example Milli-Q^{®} water obtaining a mixture; the mixture is magnetically stirred for 30 minutes and then the pH is adjusted to 4.75 ± 0.05 using NaOH or HCl 1 M; then a stirring phase for at least 30 minutes occurs obtaining a substrate emulsion. Then 30 mL of the thus obtained substrate emulsion and 2 mL of a KPi buffer (0.1 M, pH 7.0) are mixed in a titration beaker and the resulting emulsion is thermostatted at 30°C under mechanical stirring, obtaining the test aqueous environment. After the test aqueous environment is prepared, a certain quantity of an enzymatic preparation immobilized on rice husks is added to the test aqueous environment and a pH-stat titration is performed, using NaOH 0.1 M as titrating solution. The reaction is followed for 14 minutes and the hydrolytic activity, that is the enzyme activity is calculated as for the formula eq.3: $Activity \left(\frac{U}{g}\right) = \frac{{V}_{NaOH} \left(mL\right) \times \left[NaOH\right] \left(M\right)}{{t}_{Assay} \left(min\right) \times {g}_{enzyme}}$ In which:
- V_{NaOH} is the volume of the NaOH 0.1 M titrating solution added to the test aqueous environment during the assay;
- [NaOH] is the molar concentration of titrating NaOH solution used;
- g_{enzyme} is the weight of immobilized enzyme used in the reaction;
- t_{Assay} is the total time of the assay expressed in minutes.

EXAMPLE 5A: Immobilization of lipase from Thermomyces lanuginosus (TLL) on milled rice husk, with a loading of 2 mg of protein (415 U) per gram of dry of carrier, that is rice husks. Enzyme of class lipase from Thermomyces lanuginosus (TLL), in the following indicated as TTL, as a commercially available solution was provided to be used as agent to be transported by the rice husks. In detail a commercially available solution containing lipase from Thermomyces lanuginosus (TLL) with a protein content 26 mg/ml was used. An example of a commercially available solution is the product named Lipolase^{®} 100L from Novozymes^{®}. In this case, a quantity of 0.1 mL of commercially available solution containing lipase from Thermomyces lanuginosus (TLL) was diluted with demineralized water to reach a protein concentration of 1.6 mg/mL with obtainment of an agent solution in the form of an aqueous TTL solution. The obtained concentration was checked with a Bradford test. Rice husk machined to obtain a particle size in the range 200-400 micron were provided (Fig. 1, Fig. 2). A quantity of 400 mg of rice husks was wetted with 500 micro-liters of the agent solution in the form of aqueous TTL solution obtaining a wet composition of rice husk and agent solution of TTL. The resulting wet composition was mechanically mixed to form homogeneously hydrated rice husk particles, that is a mixture of wet rice husks particles with no distinct liquid phase visible and the water, once adsorbed onto the rice husk surface or absorbed penetrating into the cavities, tunnels and pores of the carrier material, hydrates the material and the agent so that no bulk water is present. This makes the method different from known ones. The resulting mixture of wet rice husk particles was dried in order to gently remove only those water molecules which are not strongly bound on the support or the agent. Drying was performed by spreading the resulting rice husk fragments on a drying surface and performing a drying procedure on the air at a drying temperature for a certain drying time. In this example, drying temperature was 25°C and drying time was 24 hours. After the drying procedure distinct fragments of rice husk containing the enzyme were obtained, together with residual water molecules hydrating the support and the enzyme, corresponding to about 9% w/w with respect to the weight of the distinct fragments of rice husk containing the enzyme. The drying procedure is provided to last for at least a minimum drying time in order to allow the creation of interactions between a lignocellulosic matrix of rice husks and the enzyme. No aggregates were observed. After drying, the distinct fragments of rice husk containing the enzyme were subjected to a washing step in which distinct fragments of rice husk containing the enzyme were resuspended in 5 mL of water and incubated under orbital mixing for 10 minutes to remove the protein particles, that is the enzyme particles, not firmly attached on the material, that is on the distinct fragments of rice husk. The supernatant is removed by decanting. The washing procedure was repeated one further time. The washed rice husk containing the immobilized enzyme was dried once again. The final drying step was performed spreading the resulting rice husk fragments on a drying surface and performing a drying procedure on the air at a drying temperature for a certain drying time. In this example, drying temperature was 25°C and drying time was 24 hours Prior art methods that make use of high temperature or solvents can promote enzyme inactivation, besides interfering with hydrogen bonds created between the agent and the rice husk. After the drying procedure obtaining distinct fragments of rice husk containing the enzyme were obtained together with water molecules hydrating the rice husk and the enzyme, in which water molecules corresponds to about 9% w/w with respect to the weight of the distinct fragments of rice husk containing the enzyme. The distinct fragments of rice husk containing the enzyme were stored at 5°C. The hydrolytic activity of the distinct fragments of rice husk containing the lipase from Thermomyces lanuginosus (TLL) was evaluated according to the tributyrin hydrolysis assay as previously explained with reference to the HYDROLYTIC ACTIVITY EVALUATION PROCEDURE, in which a quantity of 30 mg of rice husk containing the lipase from Thermomyces lanuginosus (TLL) is added to the test aqueous environment and a pH-stat titration is performed, using NaOH 0.1 M as titrating solution. The reaction is followed for 14 minutes and the HYDROLYTIC ACTIVITY, that is the enzyme activity is calculated as for the previously indicated formula eq.3. The resulting hydrolytic activity of the distinct fragments of rice husk containing the lipase from Thermomyces lanuginosus (TLL) was 619±48 U per g of dry of immobilized lipase (average of three measurements). As can be observed, the lipase from Thermomyces lanuginosus increases its hydrolytic activity upon immobilization, most probably because it undergoes an interfacial activation. This suggest that the surface of the rice husk provides a favorable environment for preserving the open conformation of lipase from Thermomyces lanuginosus. The same activation phenomenon was observed also when 1mg of lipase from Thermomyces lanuginosus (208 U) was loaded on 1 g of rice husk, obtaining a final hydrolytic activity of 409 ± 33 U per g of dry of immobilized lipase (average of three measurements).

EXAMPLE 5B: Immobilization of lipase from Thermomyces lanuginosus (TLL) on milled rice husk, with a loading of 2 mg of protein (415 U) per gram of dry of carrier, that is rice husks, where the amount of rice husk used for the immobilization is 120 g. The same procedure of EXAMPLE 5A was applied for scaling up the immobilization procedure, increasing proportionally all the volumes of the solutions and the amount of enzyme. In detail a commercially available solution containing lipase from Thermomyces lanuginosus (TLL) with a protein content 26 mg/ml was used. An example of a commercially available solution is the product named Lipolase^{®} 100L from Novozymes^{®}. In this case, a quantity of 10 mL of commercially available solution containing lipase from Thermomyces lanuginosus (TLL) was diluted with demineralized water to reach a protein concentration of 1.6 mg/mL with obtainment of an agent solution in the form of an aqueous TTL solution. The obtained concentration was checked with a Bradford test. Rice husks machined to obtain a particle size in the range 200-400 micron were provided (Fig. 1, Fig. 2). A quantity of 120 g of rice husks was wetted with 150 mL of the agent solution in the form of aqueous TTL solution obtaining a wet composition of rice husks and agent solution of TTL. The rice husk fragments were wetted with the agent solution in the form of aqueous TTL solution obtaining a wet composition of rice husk and agent solution of TTL. The resulting wet composition was mechanically mixed to form homogeneously hydrated rice husk particles, that is a mixture of wet rice husk particles with no distinct liquid phase visible and the water, once adsorbed onto the rice husk surface or absorbed penetrating into the cavities, tunnels and pores of the carrier material, hydrates the material and the agent so that no bulk water is present. This makes the method different from known ones. The resulting mixture of wet rice husks particles was dried in order to gently remove only those water molecules which are not strongly bound on the support or the agent. Drying was performed by spreading the resulting rice husk fragments on a drying surface and performing a drying procedure on the air at a drying temperature for a certain drying time. In this example, drying temperature was 25°C and drying time was 24 hours. After the drying procedure distinct fragments of rice husk containing the enzyme were obtained, together with residual water molecules hydrating the support and the enzyme, corresponding to about 9% w/w with respect to the weight of the distinct fragments of rice husk containing the enzyme. The drying procedure is provided to last for at least a minimum drying time in order to allow the creation of interactions between a lignocellulosic matrix of rice husks and the enzyme. No aggregates were observed. After the drying procedure, the rice husk particles containing the immobilized enzyme were divided into two aliquots, where only one aliquot was subjected to the washing procedure as described in EXAMPLE 5A, aimed at the removal of the protein particles, that is the enzyme particles, not firmly attached on the material, that is on the distinct fragments of rice husk. In this case the aliquot was washed by resuspending the immobilized enzyme in 500 mL of distilled water, stirring for 10 minutes at 25°C, then removing the supernatant water by filtering the rice husk with immobilized enzyme on a sieve with mesh size of 125 micron. At the end of the washing procedure, the supernatant was removed by decanting. The washing procedure was repeated one further time. The washed rice husk containing the immobilized enzyme was dried once again. Drying was performed by spreading the resulting rice husk fragments on a drying surface and performing a drying procedure on the air at a drying temperature for a certain drying time. In this example, drying temperature was 25°C and drying time was 24 hours. Prior art methods that make use of high temperature or solvents can promote enzyme inactivation, besides interfering with hydrogen bonds created between the agent and the rice husk. After the drying procedure distinct fragments of rice husk were obtained containing the enzyme together with residual water molecules hydrating the rice husk and the enzyme, corresponding to about 9% w/w with respect to the weight of the distinct fragments of rice husk containing the enzyme after the drying procedure. The distinct fragments of rice husk containing the enzyme were stored at 5°C. The hydrolytic activity of the distinct fragments of rice husk containing the lipase from Thermomyces lanuginosus (TLL) was evaluated according to the tributyrin hydrolysis assay. The hydrolytic activity of this first aliquot resulted to be 1089 ± 84 U/g. The second aliquot of TLL immobilized on rice husk was stored at 5°C without performing any further washing or drying procedure. The hydrolytic activity of this second aliquot resulted to be 2492 ± 144 U/g. The higher hydrolytic activity is ascribable to the absence of the washing step where the exposure of lipase TLL to water promotes the change of the lipase conformation towards a close form that hinders the access of the substrate to the active site of the enzyme. The two aliquots of immobilized TLL were stored under the same conditions for 8 months and their hydrolytic activity was assessed at regular intervals. That was accomplished in order to evaluate the effect of the washing procedure on the enzyme activity and verify the necessity of the washing procedure for detaching the enzyme particles not-firmly attached on the support.

FIRM IMMOBILIZATION OF LIPASE TLL WHEN IMMOBILIZED ON RICE HUSK: the firm immobilization of lipase TLL when immobilized on rice husk according to EXAMPLE 5A (400 mg scale of immobilization) and EXAMPLE 5B (120g scale of immobilization) is demonstrated by the absence of free active enzyme in the product of the interesterification. Fig. 17 is a table showing Lipase TLL released during 8 interesterification cycles, assessed by measuring the hydrolytic activity present in the raw reaction product obtained from the interesterification reaction (EXAMPLE 9) of triolein and tristearin using TLL immobilized as described in EXAMPLE 5B. The assay aims at detecting whether the interesterification product has been contaminated by free enzymatic particles that detach from the rice husk during the process and the recycling procedure. For the measurement of the hydrolytic activity in the reaction mixture, 50 micro liters of product were withdrawn at the end of each interesterification cycle (24h each) and this sample was tested in aqueous emulsion to detect hydrolytic activity towards tributyrin as described in the HYDROLYTIC ACTIVITY EVALUATION PROCEDURE. The absence of detectable hydrolytic activity in all the product mixtures assessed (Fig. 17), indicates that the active enzyme does not detach from the support over 8 cycles at 70°C, corresponding to a total of 192 h of operational use of the immobilized biocatalyst.

EXAMPLE 6: Immobilization of lipase B from Candida antarctica (CaLB) on milled rice husk, with a loading of 14.9 mg/g dry of carrier, that is rice husk. Enzyme of class lipase B from Candida antarctica (CaLB), in the following indicated as CaLB, as a commercially available solution was provided to be used as agent to be transported by the rice husk. In detail a commercially available solution containing lipase B from Candida antarctica (CaLB) with a protein content 38 mg/ml was used. In this case, a quantity of 0.1 mL of commercially available solution containing lipase B from Candida antarctica (CaLB) was diluted with demineralized water to reach a protein concentration of 11.9 mg/mL with obtainment of an agent solution in the form of an aqueous solution of lipase B from Candida antarctica (CaLB). The obtained concentration was checked with a Bradford test. Rice husk machined to obtain a particle size in the range 200-400 micron were provided (Fig. 1, Fig. 2). A quantity of 400 mg of rice husk was wetted with 500 micro-liters of the agent solution in the form of aqueous solution of lipase B from Candida antarctica (CaLB) obtaining a wet composition of rice husk and agent solution of CaLB. The resulting wet composition was mechanically mixed to form homogeneously hydrated rice husk particles, that is a mixture of wet rice husks particles with no distinct liquid phase visible and the water, once adsorbed onto the rice husk surface or absorbed penetrating into the cavities, tunnels and pores of the carrier material, hydrates the material and the agent so that no bulk water. This makes the method different from known ones. The resulting mixture of wet rice husks particles was dried in order to gently remove only those water molecules which are not strongly bound on the support or the agent. Drying was performed by spreading the resulting rice husk fragments on a drying surface and performing a drying procedure on the air at a drying temperature for a certain drying time. In this example, drying temperature was 25°C and drying time was 24 hours. After the drying procedure distinct fragments of rice husk containing the enzyme were obtained, together with residual water molecules hydrating the support and the enzyme, corresponding to about 9% w/w with respect to the weight of the distinct fragments of rice husk containing the enzyme, after the drying procedure. The drying procedure is provided to last for at least a minimum drying time in order to allow the creation of interactions between a lignocellulosic matrix of rice husks and the enzyme. No aggregates were observed. After drying, the distinct fragments of rice husk containing the enzyme were subjected to a washing step in which the distinct fragments of rice husk containing the enzyme were resuspended in 5 mL of water and incubated under orbital mixing for 10 minutes to remove those protein particles, that is the enzyme particles, not firmly attached on the material, that is on the distinct fragments of rice husk. The supernatant is removed by decanting. The washing procedure was repeated one further time. The washed rice husk containing the immobilized enzyme was dried once again. Drying was performed spreading the resulting particles on a drying surface and performing a drying procedure on the air at a drying temperature for a certain drying time. In this example, drying temperature was 25°C and drying time was 24 hours Prior art methods that make use of high temperature or solvents can promote enzyme inactivation, besides interfering with hydrogen bonds created between the agent and the rice husk. After the drying procedure distinct fragments of rice husk containing the enzyme were obtained together with some residual water in which residual water was about 9% w/w with respect to the weight of the distinct fragments of rice husk containing the enzyme. The distinct fragments of rice husk containing the enzyme were stored at 5°C. The hydrolytic activity of the distinct fragments of rice husk containing the lipase B from Candida antarctica (CaLB) was evaluated according to the tributyrin hydrolysis assay as previously explained with reference to the HYDROLYTIC ACTIVITY EVALUATION PROCEDURE, in which a quantity of 30 mg of rice husk containing the lipase B from Candida antarctica (CaLB) is added to the test aqueous environment and a pH-stat titration is performed, using NaOH 0.1 M as titrating solution. The reaction is followed for 14 minutes and the HYDROLYTIC ACTIVITY, that is the enzyme activity is calculated as for the previously indicated formula eq.3. The resulting hydrolytic activity of the distinct fragments of rice husk containing the lipase B from Candida antarctica (CaLB) was 269±17 U per g of dry of immobilized lipase (average of three measurements). As can be observed, the lipase B from Candida antarctica (CaLB) does not increase its hydrolytic activity upon immobilization, most probably because it does not undergo interfacial activation, and does not change its conformation when exposed to hydrophobic environment. When 1mg of CaLB protein (324 U) was loaded on 1 g of rice husk, the measured final hydrolytic activity of the immobilized lipase B from Candida antarctica (CaLB) was 108 ± 6 U per gram of dry of immobilized enzyme, average of three measurements.

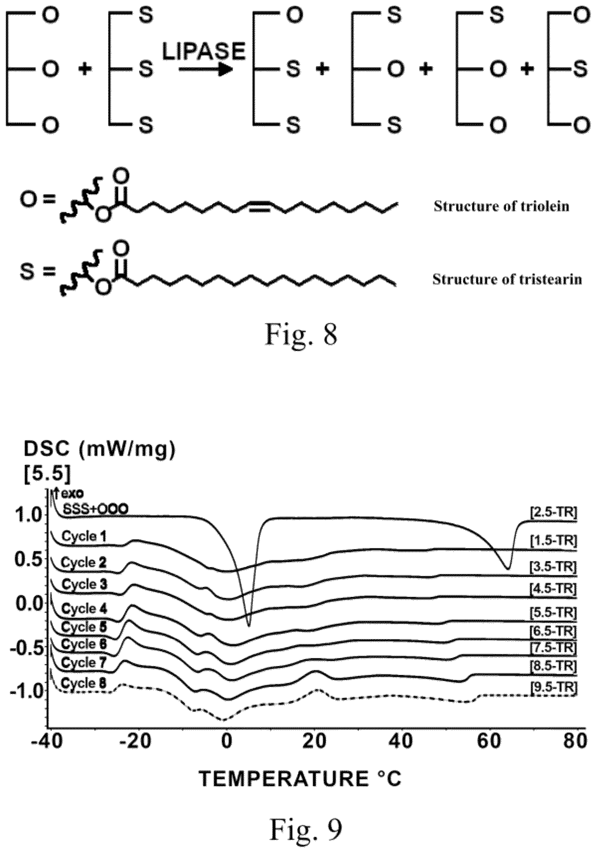

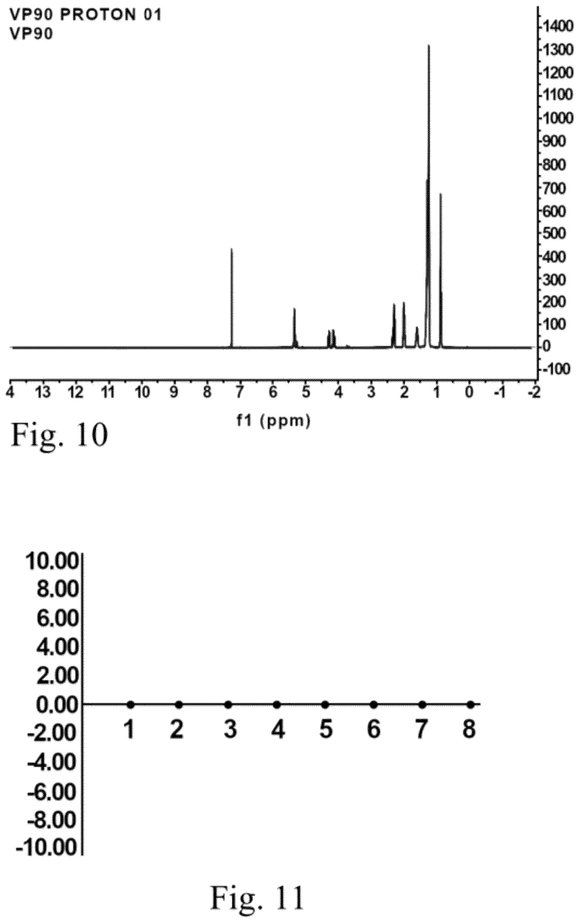

EXAMPLE 7: Application of lipase from Thermomyces lanuginosus (TLL) immobilized on rice husk according to example 5A in the interesterification between triglycerides in the form of triolein and tristearin in a neat organic reaction mixture composed by the reactants. A quantity of 0.135 g of tristearin and a quantity of 0.365 g of triolein were introduced in a round bottomed flask together with 0.05 g (31 U) of rice husk with immobilized enzyme according to example 5A with a loading of 2 mg of lipase from Thermomyces lanuginosus (TLL) / g of carrier, that is rice husk. The reaction system was mixed at 70°C for 24 hours obtaining a reaction mixture according to the reaction reported in Fig. 8, in which "O" indicates an oleic acid side chain structure and "S" indicates a stearic acid side chain structure. Triolein is the triglyceride of oleic acid (glycerol backbone with three O [= 3 oleic acid side chain]), while tristearin is the triglyceride of stearic acid (glycerol backbone with three S [= 3 stearic acid side chains]). The rice husk with immobilized enzyme still attached was separated from the reaction mixture. The mixture remaining after removing the rice husk with immobilized enzyme from the reaction mixture was analyzed by means of Differential Scanning Calorimetry (DSC) to monitor the conversion of tristearin by observing the corresponding melting peak between 50 and 70°C. The rice husk with immobilized enzyme was employed in 8 consecutive synthetic cycles, without washing between the cycles. After 8 synthetic cycles, the enzyme was analyzed in a hydrolytic assay according to the previously reported HYDROLYTIC ACTIVITY EVALUATION PROCEDURE to assess the residual enzymatic activity. The results are reported in Fig. 9, showing DSC thermograms of the raw products of the interesterification between triolein and tristearin after each synthetic cycle. The reference is a mixture of tristearin and triolein in the same ratio used for the assay. Conditions: 0.5 g of substrates, 50 mg of biocatalyst consisting in TLL immobilized on rice husk with a loading of 2 mg of protein (415 U) per g of dry carrier, that is rice husks. With reference to Fig. 9, the reaction was monitored by observing the melting peak between 50 and 70°C, corresponding to unreacted tristearin. It is evident how the preparation maintains excellent synthetic ability for 6 cycles, where the tristearin peak is barely detectable, and good synthetic ability for the remaining two cycles. The enzymatic preparation displays good synthetic abilities despite the high reaction temperature and prolonged times of operation. The formation of the products was confirmed by means of ESI-MS. No free carboxylic acid was detected in the final reaction mixture, as confirmed by NMR spectrum reported in Fig. 10, showing 1H NMR Spectrum of the sample obtained from the interesterification. In the area above 8 ppm, it can be observed the absence of signals corresponding to carboxylic acid protons. Finally, 30 micro-liters of the raw reaction mixture recovered after each synthetic cycle of the enzymatic interesterification and after removal of the rice husk with immobilized enzyme was also used as in an assay for the hydrolysis of tributyrin according to the previously reported HYDROLYTIC ACTIVITY EVALUATION PROCEDURE to determine the presence of leached enzyme in the product mixture. The results are reported in Fig. 11, showing the detected hydrolytic activity for the raw reaction mixture recovered after each synthetic cycle of the enzymatic interesterification and after removal of the rice husk with immobilized enzyme. No significative hydrolytic activity was detected, further indicating the firm binding of the enzyme on the support. The measured hydrolytic activity of the rice husk with immobilized enzyme after 8 interesterification recycles was 56 U/g of dry; the enzyme retains 9% of the initial immobilized activity (619 U/g of dry).

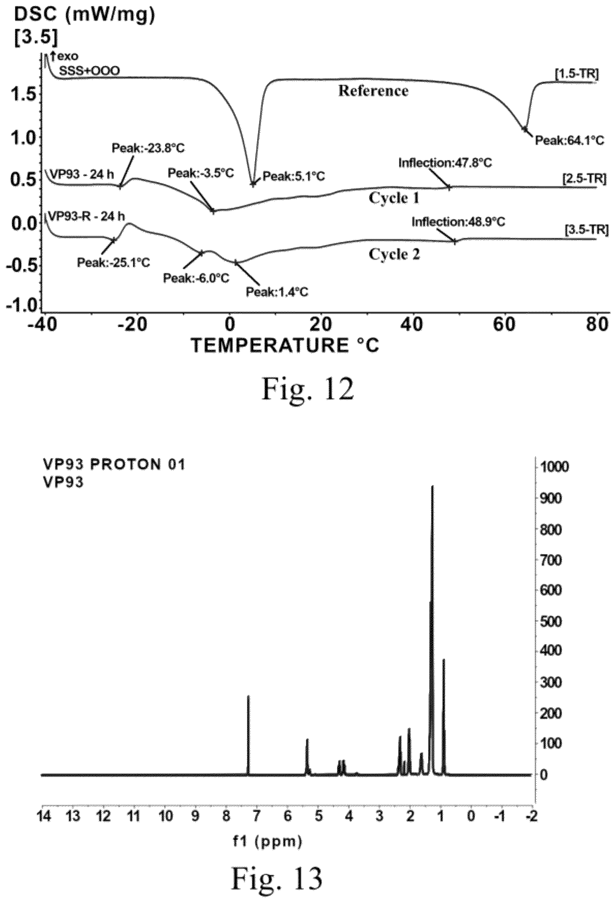

EXAMPLE 8: Application of lipase from Thermomyces lanuginosus (TLL) immobilized on rice husk according to example 5A but with a loading of 1 mg of lipase from Thermomyces lanuginosus (TLL) per g of dry of carrier in the interesterification between triolein and tristearin in a neat organic reaction mixture composed by the reactants. A quantity of 0.135 g of tristearin and a quantity of 0.365 g of triolein were introduced in a round bottomed flask together with 0.05 g (16 U, tributyrin assay) of immobilized lipase from Thermomyces lanuginosus (TLL) on rice husk according to example 5A but with a loading of 1 mg of TTL / g of carrier, (final hydrolytic activity of 409 ± 33 U per g of dry of rice husk with immobilized enzyme). The reaction system was mixed at 70°C for 24 hours. The rice husk with immobilized enzyme still attached was separated from the reaction mixture. The mixture remaining after removing the rice husk with immobilized enzyme from the reaction mixture was analyzed by means of Differential Scanning Calorimetry (DSC) to monitor the conversion of tristearin by observing the corresponding melting peak between 50 and 70°C. The rice husk with immobilized enzyme was employed in two consecutive synthetic cycles, without washing between the cycles. The results are reported in Fig. 12, showing DSC analysis of the interesterification of tristearin and triolein with TLL immobilized on rice husk. The first curve indicated with the label "reference" is a thermogram relative to the starting mixture, that is it is not a curve referred to a cycle but the reference tristearin and triolein mixture before the interesterification reaction. The second curve indicated with the label "cycle 1" is relative to the first synthetic cycle. The third curve indicated with the label "cycle 2" is relative to the second synthetic cycle performed after the first cycle without washing between the cycles. Conditions: 0.5 g of substrates, 50 mg of biocatalyst consisting in TLL immobilized on rice husk with a loading of 1 mg of protein (208 U) per g of dry carrier. As can be seen in Figure 12, the interesterification of tristearin and triolein was completed after 24 hours. The rice husk with immobilized enzyme was recycled for a second interesterification cycle, and also in this case the reaction was completed after 24 hours (see Fig. 12, thermogram of cycle 2). The formation of the products was confirmed by means of ESI-MS. No free carboxylic acid was detected in the final reaction mixture, as confirmed by NMR spectrum reported in Figure 13, showing. 1H NMR Spectrum of the sample obtained from the interesterification. In the area above 8 ppm, it can be observed the absence of peaks corresponding to carboxylic acid protons.

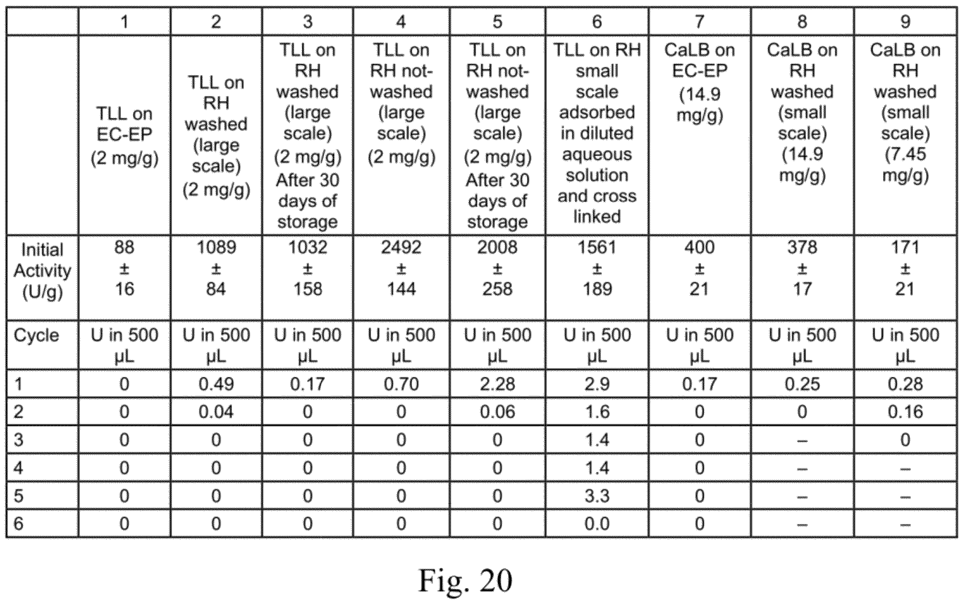

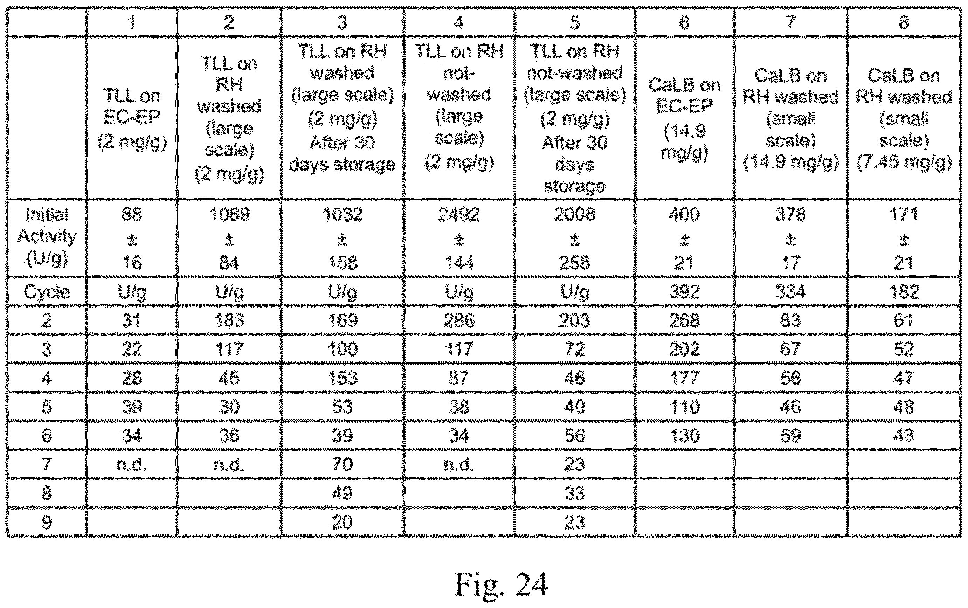

EXAMPLE 9: Application of TLL and CaLB immobilized on rice husk according to examples 5 and 6 for the hydrolysis of a triglyceride in aqueous environment forming an emulsion. The lipases TLL and CaLB immobilized according to examples 5 and 6, with two different enzymatic loadings were employed in the hydrolysis of a triglyceride, namely tributyrin, in an aqueous environment composed by a buffer, an emulsifier (Arabic gum) and tributyrin as for the previously reported HYDROLYTIC ACTIVITY EVALUATION PROCEDURE. It was provided a sequence of subsequent cycles of use of the TLL and CaLB immobilized on rice husk, in which each hydrolytic cycle was followed by washing/drying steps. The results of the subsequent cycles are reported in the table reported in Fig. 20 and Fig. 24. Fig. 24 shows the % of recovered enzymatic activities, referred to the original Units loaded on the rice husk, that is, Hydrolytic activity of TLL (A) and CALB (B) immobilized on rice husk used in aqueous emulsion for the hydrolysis of tributyrin and recovered activity upon recycling. Each cycle implies 14 min of incubation at 30 °C. Fig. 20 shows Enzymatic U detected after each hydrolytic cycle as a result of the detachment of the protein from the carrier, that is the assessment of the leaching of the active enzyme from the support when the immobilized lipases TLL (A) and CALB (B) immobilized on rice husk are exposed to an aqueous emulsion. Hydrolytic activity was measured in 500 microL of aqueous emulsion obtained after 14 min of incubation for different subsequent incubation cycles. EC-EP are commercial methacrylic resins functionalyzed with epoxy groups able to establish covalent bonds with the amino groups of enzymes and proteins in general. Fig. 18A is a microscope image of the TLL immobilized on rice husk before the interesterification process of example 9 is carried out and Fig. 18B is a microscope image of the TLL immobilized on rice husk after the interesterification process of example 9 is carried out. Fig. 18A and Fig. 18B demonstrate that the rice husks with immobilized enzyme maintain the original morphology after 192 h of operational application (8 cycles of 24 h) at 70°C (EXAMPLE 9).

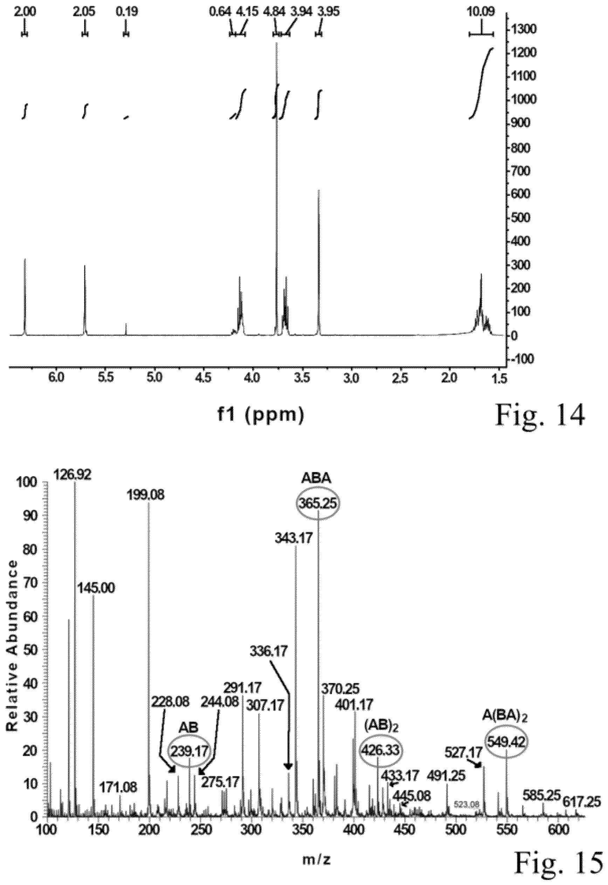

EXAMPLE 10: Application of lipase B from Candida antarctica (CaLB) immobilized on rice husk according to example 6 in the solvent free polycondensation of 1,4-butanediol and dimethyl itaconate. A quantity of 0.3954 g of dimethyl itaconate (2.50 mmol) and a quantity of 0.1126 g of 1,4-butanediol (1.25 mmol) were introduced in a 25 mL round bottomed flask together with 254 mg (50% w/w; 68 U) of lipase B from Candida antarctica (CaLB) immobilized on rice husk according to example 6 with a loading of 14.9 mg / g of carrier. The system was mixed on a rotary evaporator at a temperature of 50°C; the pressure was set at 500 mbar, and then reduced at 100 mbar after 1 hour of mixing. The total reaction time was 72 hours. The raw reaction product was dissolved in 30 mL of dichloromethane. Then the rice husks with immobilized enzyme were recovered by filtration. The remaining of the raw reaction product after filtration was recovered by evaporation of the dichloromethane under reduced pressure. The recovered reaction product was then analyzed by 1H NMR in CDCl3 and by ESI-MS spectrometry. The results of the analysis are reported in Fig. 14, showing the 1H NMR spectrum in chloroform-d (CDCl3) of the recovered reaction product [1H NMR spectrum (400 MHz, CDCl3) δ 6.32 (s), 5.71 (s), 4.17 - 4.08 (m), 3.76 (d), 3.72 - 3.63 (m), 3.33 (d), 1.80 - 1.57 (m)] with superimposed the curves of integration of each peak from the spectrum with corresponding numeric values of the areas. The spectrum was analyzed by comparison with literature [A. Pellis, L. Corici, L. Sinigoi, N. D'Amelio, D. Fattor, V. Ferrario, C. Ebert and L. Gardossi, Green Chem., 2015, 17, 1756-1766]. Comparison with literature confirmed the correct attribution of NMR peaks and therefore the obtainment of the desired reaction product. The two signals at 6.32 and 5.71 ppm are characteristics of the =CH2 group of the itaconic portion of the polymer. The formation of polycondensation products is evidenced by the multiplet at 4.17-4.08 ppm. Figure 15 reports the ESI-MS spectrum of the recovered reaction product. Labeled ESI-MS positive ion mass spectrum of the polycondensation products of dimethyl itaconate (A) and butanediol (B) catalyzed by lipase B from Candida antarctica (CaLB) immobilized according to example 6. The labelled adducts were identified in accordance with literature. The presence of said adducts evidences the success of the polycondensation reaction.

### THE INVENTIVE METHOD

Considering prior art studies on the possible use of different carries, there is a teaching away pointer with respect to the use of rice husks because prior art studies evidenced that when working in diluted aqueous media the affinity of proteins for the rice husk surface is low, so that previous studies reported low loadings of enzymes on rice husk. Such studies also indicated that when the protein is immobilized on rice husk starting from a diluted solution, even when binders are added, the protein detach promptly from the support if exposed to aqueous media.

Thanks to the method according to the present invention, a higher loading of the enzymatic protein employed in the immobilization of enzymes on rice husk and firm binding are achieved, contrary to the disclosure of prior art studies, which generally implies the incubation of the solid support in a diluted protein solution, where the partition of the enzyme from the aqueous phase unto the support is requested. Indeed, the present method does not use a classical diluted liquid enzymatic solution, but spreads a small volume of enzymatic solution directly on the carrier, thus forcing the enzyme to enter into close contact with the rice husk surface and enter rice husk cavities, tunnels and pores. This is possible because of the high level of water retention of rice husk (40% w/w). Upon a slow and gentle removal of the water molecules which are not strongly bound on the enzyme or the rice husk, the enzyme establishes strong interactions with the carrier and it is partially trapped into the cavities, tunnels and pores. This is evident from the fact that the immobilized biocatalyst displays enzymatic activity after extensive washing with aqueous buffer or prolonged use in aqueous media. The slow removal of the residual water (about 24h) under mild conditions (e.g. atmospheric pressure and room temperature) is necessary for establishing the interactions: it was verified that there is no significant protein leaching from the support, as shown, for example, with reference to Fig. 20, which is a table showing the Enzymatic U detected after each hydrolytic cycle as a result of the detachment of the protein from the carrier with reference to example 9, that is the assessment of the leaching of the active enzyme from the support when the immobilized lipases TLL (A) and CALB (B) immobilized on rice husk according to examples 5 and 6 are exposed to an aqueous emulsion.

The present invention dispels the dogma that immobilized enzymes for industrial applications must have a high loading of protein or enzymes on a small volume of carrier, so to express a high activity. The negative consequences of prior art approach are mainly use of large amount of enzymatic protein in the immobilization procedure with a large waste in terms of Units expressed after immobilization and lower mass transfer because of reduced surface of the immobilized biocatalyst.

On the contrary, the present method distributes a small amount of enzymatic protein on a large surface of rice husk, so that a larger volume of carrier is used in the process and this leads to a more efficient mass transfer and higher retention of enzymatic activity. This approach is possible thanks to specific properties of rice husk, that is low density (<0.5g/mL) so that on a weight basis a larger surface is available for the protein distribution.

From the examples reported above, it is clear that the inventive method for immobilization of one or more agents on a carrier comprising rice husks provides (Fig. 1) the following steps:
(A) Providing rice husks;
(B) Checking if a pre-treatment (C) is needed on the provided rice husks, and in case perform a pre-treatment (C) procedure on the provided rice husks;
(D) Providing one or more agents to be transported by the rice husks particles;
(E) Checking if the one or more agents to be transported by the rice husks particles are provided in the form of a liquid phase or in the form of a liquid solution and, if not agents' solution preparation (F) in a liquid state; in any case a checking of the concentration (G) is performed with possible adjustment (H) of the concentration of the agents in the agents or agents' solution in a liquid state in which the agent or agents' concentration is in a required range for the specific application;
(L) Wetting the rice husks particles with the agent or agents' solution with obtainment of a wet composition in such a way that there is no separate liquid phase at the end of the mixing procedure, that is after the successive mixing step (M), so that the wet rice husk particles are not surrounded by any bulk liquid phase;
(M) Mixing the wet composition obtaining wet rice husks or rice husk particles containing the one or more agents;
(N) Drying gently the resulting wet rice husks or rice husk particles containing the one or more agents obtaining distinct fragments of rice husk containing the agents with only retained water molecules which are strongly bound to the rice husk particles and the one or more agents. The drying step removes some excess water by promoting the evaporation of those water molecules which are not strongly bound to the rice husk or the enzyme;
(P) Checking if a washing step is required and in case perform a washing step (Q) and checking (R) if an additional drying step (N) is required before storing or packaging of the product; The washing step (Q) can be repeated more than one time;
(S) Storage of the resulting product, if required, or packaging.

The operative temperature used in the method, as for example in steps (L), (M), (N), must be chosen according to the thermal stability of the agents in the solution. For example, considering agents in the form of enzymes characterized by a low thermal stability, the method can be carried out at temperatures in the range 4-10 °C, but preventing the freezing of the agents' solution. For enzymes characterized by sufficient thermal stability, as the ones reported in the examples, the method can be carried out at room temperature (e.g. 18-28 °C). With reference to the pre-treatment (C), it provides (Fig. 1) the following steps:
(C1) Checking if a pre-washing (C2) is needed on the provided rice husks and in case perform a pre-washing procedure (C2) in order to remove fines and other small size solid contaminants. In many cases of provided rice husks, the rice husk is quite clean and can be stored at room temperature in simple plastic bags for more than 10 years at temperature between 20 and 30 °C, without any degradation or contamination or attack of moulds or insects. This property is probably a consequence of the presence of the silica on the surface of rice husk. For the implementation of the inventive method, the pre-washing procedure (C2), if needed, is preferably a pre-washing with a composition of ethanol:water 1:1 to remove powders and fines from the surface.
(C3) Checking if particle size of the provided rice husks is in the required range for their use in the inventive method and in case perform a size reduction procedure (C4) in order to bring the size of the rice husks into the required size range for their use in the method.

Even if the whole rice husks with average size of 10.000 micron without any fragmentation can be used in the inventive method, it is advisable to reduce the particle size in order to allow the exploitation of the structural features of the rice husk while responding to technical requirements of most industrial processes, e.g. mass transfer, easy recovery and down-stream processing. Therefore, a preferred size range of rice husk particles is 100-1000 micron, preferably 150-800 micron, the optimal range being 200-400 micron.

The optimal range of particle size (200-400 micron) is preferable for two reasons. Most commonly used commercial resins for enzyme immobilization fall in the same size range. This is because fine particles are difficult to be removed after the process and also create pressure in column reactors. Previous experiments conducted on lower particle sizes showed reduced or no displayed activity, probably because an extensive cutting, necessary to obtain smaller size particles, destroys pores and cavities and also the tri-dimensional structure of the rice husk, which is a kind of hollow fiber allowing the enzyme to access the tunnels corresponding to the tracheids. Considering the use of whole rice husks without any fragmentation, it must be considered that whole rice husks are a kind of leaves with size in the range of 0.4-1.2 cm and "sealed" so that the inner channels and tracheids are not accessible from the outside, constituting an obstacle to penetration of the enzymes to be immobilized on the rice husk so that as a result, enzymes immobilized on whole rice husks show four times lower activity if compared to enzymes immobilized on rice husks particles having size in the optimal range of particle size. The reasons are that the channels of the tracheids are not accessible and only the superficial cavities are available.

With reference to the size reduction (C4) of the rice husks, a milling procedure can be provided. The rice husk is very robust and recalcitrant to most type of possible milling methods. That is because rice husks have a relatively high content of SiO₂ (about 15 -17 % w/w of silica) part of which is located on the surface of rice husks. The presence of an high content of silica in rice husks causes damages to most milling rotors. Our direct experience led to the damage and destruction of ball milling systems and simple blade milling systems. Therefore, the preferred milling method to reduce the size of rice husks to get a required particle size for the use in the inventive method is the use of knife rotors which are effective in reducing the dimension of the rice husks. Therefore, the rice husk is actually cut into small fragments, which retain the original tridimensional structure of the material so that the inner material is exposed but the structure does of rice husks does not collapse.

With reference to the step (D) relative to providing one or more agents to be transported by the rice husks particles, it must be underlined that even if the reported example are relative to the use of only one agent, that is enzyme, at time, it will be apparent to expert in the field that the described inventive method can provide the use of one or more agents at time because the inventive method provides an efficient immobilization of the agents on rice husks particles favoring the penetration of one or more agents at time into pores and cavities and also into the tri-dimensional structure of the rice husk which also comprises tunnels of the tracheids. In some cases, enzymes need co-factors, such as the case of oxidoreductases so that multiple agents can be immobilized on rice husks at one time with the inventive method comprising one or more enzymes together with one or more cofactors, like for example NADH, NAPH, and similar ones. In some cases, a racemase enzyme is added in reactions aimed at resolving racemic mixtures and produce enantiopure products so that multiple agents can be immobilized on rice husks at one time providing one enzyme for the reaction and an additional racemase enzyme. Another example of use of multiple agents immobilized on rice husks at one time involves the use of catalase enzyme, which degrades the H₂O₂ formed during some oxidative reactions because H₂O₂ is undesired due to its detrimental effect for the stability of enzymes. For example, consider galactose oxidase which is used to oxidize alcohol groups, causing the release of H₂O₂, which is removed by adding a catalase enzyme. Moreover, some reactions can be designed to occur as a cascade, in multiple steps in which a first agent is active to catalyze a first reaction and a second agent is active to catalyze a second reaction in which both the first agent and the second agent are immobilized on the same rice husks fragments allowing the cascade of reaction.

Purpose of the invention is to immobilize agents, like for example enzymes, on rice husk particles for successive use of the product thus obtained in subsequent processes. Disclosed examples of enzymes are glucoamylase from Aspergillus niger, lipase from Thermomyces lanuginosus (TLL), lipase B from Candida antarctica (CaLB). It will be apparent to expert in the field that any enzyme can be immobilized on rice husk particles according to the inventive method. In general, any enzyme can be immobilized on rice husk particles as for example natural (extracted or present in living organism) or obtained by genetic engineering and manipulation, belonging to the six classes defined by the International Union of Biochemistry (IUBMB): EC 1 Oxidoreductases, EC 2 Transferases, EC 3 Hydrolases, EC 4 Lyases, EC 5 Isomerases, EC 6 Ligases / synthetases. Moreover, any protein or biological macromolecule able to catalyze a chemical reaction can be immobilized on rice husk particles according to the inventive method.

Exemplary applications of the rice husks fragments with immobilized agents according to the immobilization method of the present invention are:
- products for health: the rice husk particles with firmly immobilized agents prepared according to the present invention can be used for Immobilization of enzymes for the pre-digestion of fats present in food or milk formulations used in the field of diets products, as for example immobilized lipases applied for the pre-digestion of fats for patients suffering from cystic fibrosis or other pathologies that reduce the activity of pancreatic lipases;
- the rice husk particles with firmly immobilized agents prepared according to the present invention, could be administrated orally to patients, since the rice husk is made by natural fibers and silica and is not toxic and the enzyme could be diluted more accurately in the feed and also would be more stable. Other enzymes are commercially available for oral administration but they are formulated through encapsulation, which requires addition chemical ingredients and specific equipment;
- the rice husk particles with firmly immobilized agents prepared according to the present invention, can be used for immobilization of enzymes to be added in animal feed. Enzymes are used as feed supplements for poultries and other livestock. Rice husk could be eaten by animals as a natural fiber with the advantage of having enzymes immobilized in the rice husk as a supplement for the animal diet. Rice husk is not toxic and the enzyme could be diluted more accurately in the feed and also would be more stable;
- the rice husk particles with firmly immobilized agents prepared according to the present invention, can be used for immobilization of enzymes to be used for bioremediation in different eco-systems (soil, sea, internal water, etc.). Notably: rice husk has a density of about 0.4 and it floats on the water. That makes rice husk particularly suitable for applications in case of oil spills, or dissolution of mucilage covering the sea surface, so that enzymes immobilized in the rice husk can be effectively reach their target with the benefit of not being dispersed in the water environment thanks to immobilization achieved by means of the inventive method
- the rice husk particles with firmly immobilized agents prepared according to the present invention, can be used for immobilization of protein-based vaccines for animals. The rice husk particles with firmly immobilized agents prepared according to the method of the present invention, could be used for administering protein-based vaccines for animals, as for example in the field of aquaculture to administrate protein-based vaccines to fishes. Rice husk is not toxic and the enzyme could be diluted more accurately in the feed and also would be more stable;
- the rice husk particles with firmly immobilized agents prepared according to the present invention, can be used for immobilization of enzymes to be used in fossil oil fields for improving petroleum extraction;
- the rice husk particles with firmly immobilized agents prepared according to the present invention, can be used for immobilization of bio-stimulants for agriculture;
- the rice husk particles with firmly immobilized agents prepared according to the present invention, can be used for immobilization of agent for bioaugmentation or microbiota for the soil for use in agriculture;
- the rice husk particles with firmly immobilized agents prepared according to the present invention, can be used for immobilization of nutrients as for example ammonia, phosphate, biofertilizers and biopesticides for the soil or plants for use in agriculture;
- the rice husk particles with firmly immobilized agents prepared according to the present invention can be used for Glucoamylase for starch hydrolysis and production of bioethanol or glucose syrup;
- the rice husk particles with firmly immobilized agents prepared according to the present invention can be used for Lipases for the synthesis of cocoa butter analogues and any other application in the food sector, for which the product is particularly advantageous because the enzyme carrier is natural and usable without organic solvents;
- the rice husk particles with firmly immobilized agents prepared according to the present invention can be used for ester synthesis (lubricants, emollients, surfactants, plastifiers, texturing agents).

The rice husk particles with firmly immobilized agents prepared according to the method of the present invention, were used also for new reactions, such as interesterification starting from non-edible oils. The products are new and usable as ingredient in cosmetic formulations. Rice husk particles with firmly immobilized agents in the form of TLL for oil and fats transformation for cosmetic ingredients production is particularly advantageous because the enzyme carrier is natural and usable without organic solvents.

The rice husk particles with firmly immobilized agents prepared according to the method of the present invention are particularly advantageous also due to the low cost which is a key factor because nowadays many large-scale enzymatic processes cannot be implemented because of the high cost of carriers with immobilized enzymes.

With reference to the step (E) relative to checking if the one or more agents to be transported by the rice husks particles are provided in the form of a liquid phase or in the form of a liquid solution, and in case prepare an agent solution in a liquid state it must be underlined that this is an essential step of the method because a liquid state is required in order to allow the monomolecular dispersion of the agent (i.e. solubilization) and the subsequent penetration of the one or more agents into the cavities, tunnels and pores of the carrier material establishing strong interactions between the lignocellulosic surface and the silica of the matrix of the carrier material and the agents, as, for example, enzyme or protein or another agent selected from the previously reported list of possible agents to be used. With reference to the preparation of the agent solution in a liquid state with an agents' concentration in a required range, the required range obviously depends on the specific application for which the rice husks with one or more agents are prepared. The final specific application or use determines the amount of agents to be uploaded on the carrier. Therefore, the dilution of the agents' solution is made according to the process technical requirements, i.e. the amount of agents needed to perform the reaction in case of enzymes or the amount of supplements to achieve a dietarian target in animal feed or the amount of bio-stimulants, nutrients, biofertilizers, biopesticides, microbiota for the soil or plants for use in agriculture. As a general principle, the required range for the agents' concentration in the wetting solution is such that after the drying step (N), a loading of one or more agents on rice husks particles is obtained in which the agent's content is in the range of 0.4-150 mg per gram of dry carrier, that is rice husks particles, preferably in the range of 1-100 mg per gram of dry carrier, that is rice husks particles. In principle a saturated agents' solution can be used and then dilute the solution as it is convenient for the final application. The required range for the agents' concentration in the wetting solution has an upper limit which depends on the solubility of the agents in the solution. As experts in the field know, solubility of agents in a solution differs among the different type of agents. Even considering the class of agents comprising enzymes, different enzymes have different solubility. The required range for the agents' concentration in the wetting solution has not a lower limit. Concerning the lower limit of the range, the agents' concentration in the wetting solution could even be lower than 0.4 mg/mL or 0.8 mg/mL depending on the specific agent. But in case a very low loading of agents, as for example enzyme or protein, is required, like for example in the case of the preparation of micronutrients for animals, there is no impediment for diluting the protein solution to even lower concentrations.

With reference to the wetting step (L), the solid particles are wetted with the aqueous solution of the agents, either buffered or not buffered. Wetting of the rice husk was performed by depositing dropwise the enzyme/water mixture on the rice husk. The procedure was performed at room temperature (25°C). One important consideration is that the rice husks in the wetting step (L) should be wet in such a way that there is no residual, separate, distinct liquid phase at the end of the mixing procedure, that is after the successive mixing step (M), so that the wet rice husk particles are not surrounded by any liquid phase. This means that most of the agents' solution has been adsorbed on the rice husk surface or absorbed penetrating into the cavities, tunnels and pores of the carrier material, that is rice husks particles. After the drying step (N) those water molecules which are not strongly bound on the carriers and the enzyme are gently removed in order to reach a maximum water content of about 15% w/w with respect to the weight of the distinct fragments of rice husk containing the enzyme, preferably less than about 10% w/w with respect to the weight of dry distinct fragments of rice husk containing the enzyme, after the drying procedure, that is after step (N). In principle, 125 micro-liters is the volume of aqueous solution that optimally wets 100 mg of rice husk with a particle size in the range 200-400 micron, which corresponds to the quantity of 500 micro-liters of aqueous solution / 400 g of rice husk disclosed in the examples. This depends by the water absorption capacity of rice husk pre-treated as described. There might be some minor variations of water absorption capacity as a function of the particle size or the variety of the rice plant (e.g. Asian origins). Therefore, an applicable range of the volume of aqueous solution with respect to the quantity of rice husk particles to be wet is between 20 micro-liters of aqueous solution / 100 mg of rice husk particles and 200 micro-liters of aqueous solution / 100 mg of rice husk particles, preferably between 50 micro-liters of aqueous solution / 100 mg of rice husk particles and 170 micro-liters of aqueous solution / 100 mg of rice husk particles.

It is important to underline that the described method differs from known methods of prior art as for example:
- conventional known protocols employing larger volumes of diluted liquid solutions of proteins, where the carriers are placed for establishing interactions with the solid material; in the disclosed method of the present invention no large volumes of diluted liquid solutions is required because the invention provides a method in which the result is an efficient immobilization of the agents, as for example enzyme or proteins, inside cavities, tunnels and pores of the carrier material establishing strong interactions between the lignocellulosic surface and the silica of the matrix of the carrier material and the enzyme or protein;
- deposition method which implies the precipitation of a protein from a solution to cover a solid support because the invention provides a method in which the result is an efficient immobilization of the agents, as for example enzyme or proteins, inside cavities, tunnels and pores and onto the surface of the carrier material establishing strong interactions between the lignocellulosic surface of the matrix and the silica of the carrier material and the enzyme or protein preventing the loss of the agents like in the final product from deposition method in which the agents are located on the surface only of the carrier.

Therefore, it is important to make clear that no residual liquid phase persists after the mixing procedure, that is the addition of the agents by means of adsorption of the agents' solution on the surface of the carrier or absorption penetrating inside cavities, tunnels and pores of the carrier material, that is rice husks particles.

With reference to the step (M) of mixing the wet composition obtaining wet fragments of rice husk, it is important to achieve a homogeneous dispersion of the agent or agents' solution on the surface and cavities of the rice husk by mixing for a period of time between 3 and 10 minutes, preferably between 4 and 6 minutes, even more preferably 5 minutes, using any mechanical mixing method or device for the spreading of the liquid solution containing one or more agents in order to promote the homogeneous distribution of the liquid solution onto the rice husk and to avoid the formation of a distinct aqueous phase at the end of the mixing procedure. Any mechanical mixing method, employing, for instance turbines or impellers, helical ribbons or anchor mixers, or rotational mixing system is usable without compromising the structural integrity of the rice husk, due to its robustness. This feature makes rice husk different from most carriers, such as methacrylic and polyacrylic resins, which are damaged by most mechanical mixing methods, suffering from fragmentation and formation of fine particulates. The mixing is carried out at a speed such that without compromising the structural integrity of the rice husks particles is not mechanically compromised by the mixing step, due to the robustness the rice husks particles. This feature makes rice husks particles different from most carriers, such as methacrylic resins, which are damaged by most mechanical mixing methods, suffering from fragmentation and formation of fines.

The resultant moist substance in the form of an ensemble of distinct rice husk particles with immobilized agents, obtained by mixing the dry rice husk particles with the liquid solution containing one or more agents do not form a soft paste since the rice husk particles remain rigid and there is no homogeneous solid phase. The residual water molecules do not form a distinct aqueous phase since the volume of the solution containing the agents is selected in such a way to have only water molecules engaged in the hydration of the agents, adsorbed on the rice husk surface or absorbed penetrating into the pores and channels of the rice husk. The obtained mixture is an ensemble of solid wet rice husk particles without any considerable distinct liquid phase observable even if moisture is still present to be eliminated in the next drying step (N). After the drying step such particles of immobilized agents have an agents' loading in the range of 0.4-150 mg per gram of dry carrier, that is rice husks particles, preferably in the range of 1-100 mg per gram of dry carrier, that is rice husks particles. That is the morphology of the carrier, that is rice husk particles, does not change. This is also quite visible from the images (Fig. 4, Fig. 5) of the dry rice husk with particle after the application of the method according to the present invention.

With reference to the step (N) relative to drying the resulting wet rice husks or rice husk particles containing the one or more agents obtaining the distinct fragments of rice husk containing the agents and only residual water molecules bound to the enzyme and adsorbed or absorbed penetrating onto the rice husk particles, this step can be, for example, performed spreading the moist rice husk or the rice husk particles resulting from the previous step (M) on a drying surface and perform the drying phase until a maximum quantity of possible residual water is obtained which is in the range 1-20 % w/w with respect to the weight of the distinct fragments of rice husk containing the agents after the drying procedure, preferably in the range 4-15 % w/w, even more preferably in the range 7-11 % w/w, like for example 8 % w/w, 9 % w/w, 10 % w/w, 11 % w/w. The drying surface is preferably a smooth surface which for example can be selected from a glass drying surface, a ceramic drying surface, a metal drying surface. The drying surface needs to be chemically inert. Therefore, any drying surface made of a chemically inert material can be used. The drying surface can be a solid surface or a net shaped surface or a surface provided with holes to favor the drying of the mixture of wet rice husks or rice husk particles containing the one or more agents. The wet rice husks or rice husk particles containing the one or more agents are preferably spread on the drying surface with a thickness of the mixture approximatively corresponding to the thickness of a single layer of fragments. Preferably the drying step (N) occurs on the air at 25°C for 24 hours. The 24 hours phase is necessary for letting the agents' solution penetrating into the cavities, tunnels and pores of the carrier material, that is rice husk particles and for creating interactions between the agents and the lignocellulosic matrix and the silica. In general, the drying step (N) occurs at temperature comprised between 5 and 50 °C, preferably at 25°C, for a drying time in the range of 12-48 hours, preferably for 24 hours. The drying step (N) preferably occurs under environmental pressure. Ventilation can be used only under controlled conditions because rice husk is very light and it would fly. Cold flow of air or gas could help the drying when thermally sensitive enzymes are immobilized at low temperature, because in such condition, the spontaneous evaporation of water would be too slow. Moreover, nitrogen flow could be used in case of enzymes sensitive to oxidating environment.

The allowed maximum level of residual water also depends on the successive application of the obtained final product. Indeed, for enzymes to be used in aqueous environment possible residual water is preferably in the range 9-15 % w/w with respect to the weight of the distinct fragments of rice husk containing the agents after the drying procedure. In order avoid competing hydrolytic reactions for enzymes to be used in non-aqueous environment possible residual water is preferably in the range 4-9% w/w with respect to the weight of the distinct fragments of rice husk containing the agents after the drying procedure. Considering the disclosed examples, the results obtained in the reactions catalyzed by TLL indicate that the amount of water is optimal, since no free carboxylic acid was detected in the final reaction mixture, as confirmed by NMR spectrum reported in Fig. 10 relative to example 7 which disclosed the application of lipase from Thermomyces lanuginosus (TLL) immobilized on rice husk according to example 5A in the interesterification between triglycerides in the form of triolein and tristearin in a neat organic reaction mixture composed by the reactants.

Residual water is due to the fact that the rice husk particles and the agents retain some humidity even after prolonged dehydration, that is after the drying step (N), because cellulose, silica and proteins are hydrophilic polar molecules highly prone to be hydrated. Such strongly bound water molecules can be stripped only under harsh treatments that would denature the enzymes (e.g. high temperatures). The term "residual water" must be intend not as indicating a visible liquid phase residual water but as indicating residual water in the form of hydrating water, as for example water molecules bound through hydrogen bonds on cellulose or silica of the rice husk particles and bound on the agents. The residual water hydrates the enzyme and the solid matrix. It cannot be recovered.

No aggregates are produced or observed in any step of the method. This depends on the use of rice husk as a matrix, which is mechanically robust and does not undergo any further morphological modification or fragmentation during the immobilization method and it also depends on the fact that the immobilization method does not involve the use of binders to "glue" the RH fragments together, as reported in prior art methods.

In general, the presence of aggregates should be avoided because aggregates have the disadvantage of disaggregating in aqueous environment. Moreover, the presence of aggregates is undesirable because aggregates are not mechanically stable under magnetic stirring or in high viscous media, thus limiting the possible applications of the final immobilized agent. Moreover, the presence of aggregates is undesirable because aggregates prevent the substrate from accessing protein molecules that are located inside the aggregate, thus limiting the enzymatic activity. The main factor that improves the activity of the immobilized enzymes is their distribution on surfaces which must be as wider as possible, thus promoting optimal mass transfer and the access of the substrates to the enzyme active site.

As an alternative to the disclosed steps, agents, like for example enzymes, could be immobilized on solid particles of the carrier using a spay drier or fluidized bed reactor and then promoting the drying by means of an air/gas flow. However, in the inventive method a slow drying step is required, in which the term "slow" is to be intended as indicating at least 10 hours, so that during the drying step (N) the agents-rice husk particles interaction is allowed and such interactions are established so as to prevent the agents to leak off the rice husk particles. The optimal immobilization according to the inventive method requires penetration of the agents in the cavities, tunnels and pores of the carrier material, that is rice husk particles, but also adsorption penetrating on the surface of the material and the creation of interactions between the lignocellulosic matrix and the silica of rice husk particles and the agents.

The obtained product as result of the steps (L), (M), (N) is composed by the distinct fragments of rice husk particles containing the agents and only some water molecules hydrating the rice husk and the enzymes, that is the one or more agent. It is important to underline that the entire solution containing the enzyme molecules adsorbs onto the rice husk surface or absorbs penetrating into the cavities, tunnels and pores of the carrier material, thus hydrating the rice husk. No aqueous phase remains around the rice husk. To make an example of the status of the obtained ensemble of distinct rice husk particles with immobilized agents, it is similar to the status obtained adding water to sand so that the obtained ensemble of sand and water just looks like moist sand, but it's definitely a solid, and no separate water layer can be visibly seen. As explained in the examples and relative analysis the most reliable measurement to confirm the effectiveness of the disclosed method of immobilization is the enzymatic activity retained after immobilization. In general, the agents lay on a generic carrier according to methods of prior art might be adsorbed on the carrier but they could be inactive. The inventive method provides an efficient immobilization of the agents and at the same time provides a high activity of the immobilized agents.

It will be apparent to expert in the field that the agents' amount can change according the application or not of washing steps (Q) after a first drying step (N). The washing step (Q) is important for enzymes to be used in aqueous environment, whereas it is unnecessary for applications in hydrophobic media (e.g. TLL lipase catalyzed interesterification). When no additional washing step is applied, all agents used in the immobilization procedure remains on the support after the drying step (N). Some test confirmed that in some cases washing procedure is unnecessary, as for example in the case of immobilization of TLL in which, by omitting the washing step, the same performance was obtained.

In general, the washing steps (Q) provides suspension of the obtained product from step (N) in a buffer, like for example deionized water or a potassium phosphate buffer (0.1 M, pH 8), incubation under orbital mixing with a speed in the range 100-200 rpm for 5-20 minutes, preferably about 10 minutes, to remove the protein particles not firmly attached on the rice husks, removing the supernatant. Speed range above 200 rpm is not advisable to avoid enzyme stress. Removing the supernatant can be performed as a liquid phase removal by filtering or decanting, that is pouring the liquid without disturbing the solid sediment, after centrifugation in the orbital mixer.

In some cases, repeating of the washing procedure is provided, for example when the enzyme must be used in aqueous media and those enzyme molecules which are not tightly bound on the surface could be released in the reaction mixture. This is particularly important when using a very concentrated enzyme solution in the beginning, to remove excess enzyme not strongly bound on the carrier.

In the washing step (Q) of EXAMPLE 1, the use of potassium phosphate buffer (0.1 M, pH 8): is provided, for example performing a resuspension in 5 mL of the obtained product from step (N). A potassium phosphate 0.1 M buffer has a sufficient ionic strength to promote the removal of agents' particles, like for example enzymes or protein, which are deposited and not bound on the rice husk particles. Higher ionic strength could induce protein denaturation. A potassium phosphate 0.05 M buffer could also be used. The pH could be between 7 and 8. However, the pH of the buffer should be chosen also taking into account possible detrimental effects that a certain pH might cause to the agents, like enzymes. Nevertheless, pH 7 and 8 usually do not hurt proteins and enzymes stability.

Test were conducted to check the effect of the washing step. Considering the previously reported example 3, relative to glucoamylase, the amount of enzyme particles released in aqueous environment under process condition was assessed. Results indicate that no significant leaching was observed during 23 days of continuous use of the product. In the case of TLL enzyme, it was used in interesterifications in non-aqueous media (triglycerides) and recycled for 8 subsequent days at 70°C. In such hydrophobic media the enzyme retained very high activity for repeated cycles and no leaching of the enzyme was detected in the reaction mixture for the whole 192 h of exposure to operating conditions. No significant leaching, if compared to the experimental error or the protein released by a CALB covalently immobilized on a commercial epoxy functionalized resin, was observed when of the TLL and CALB lipases immobilized on rice husk were incubated in an aqueous emulsion for 14 min for 6-8 cycles. On the contrary, a massive leaching was observed over 5 cycles from lipase TLL immobilized on rice husk according conventional protocols (diluted aqueous solution and crosslinking). This indicates that the procedures described in EXAMPLES 1, 2, 4, 5A, 5B and 6 are effective to promote the firm immobilization of the enzymes on rice husk, without the need of further crosslinking agents or chemical modification of the support. The enzymes are bound on the support and do not detach from the carrier during the reaction or during the recovery and recycling. When the same TTL immobilized on rice husk according to EXAMPLE 5B was not washed with water after immobilization and drying, some minor release of enzyme was observed but only during the first cycle (out of 6 in total). The not-washed immobilized TLL expresses a two fold higher initial activity because it was not exposed to water, which favors the modification of the conformation of the lipase towards the closed and less accessible form. In conclusion: the amount of lipase molecules which are not firmly bound on the rice husk is negligible or very limited, even when the lipases immobilized on rice husk are not washed after the first drying step. Such lipase molecules can be removed only under a complex and viscous emulsion system that interact with the carrier as a kind of detergent, also causing the denaturation of the lipases. When exposed to water or hydrophobic media the lipases molecules remain firmly bound on the rice husk.

Considering the storage step (S) of the resulting product, storage characteristics depend on the final product characteristics. As for the provided examples, considering the use of glucoamylase, storage is provided to occur at 5°C suspended in 0.05M buffer pH 7 (1 g in 5mL) because glucoamylase requires to be highly hydrated and subsequently applied in aqueous media. Each enzyme must be stored according its specific features and requirements in order to maintain its activity (i.e. optimal temperature, pH, ionic strength). These data are to be considered as available in prior art as a general knowledge for expert in the field. The optimal storing conditions for the agents, like for example the native enzyme, must be applied also to the immobilized enzyme, provided that the immobilized enzyme do not have to be frozen. Also in this case, the optimal storing conditions for the native agents have to be considered as available in prior art as a general knowledge for expert in the field As an example, storage can be provided with a storage temperature in the range 3-5°C, above water freezing point because freezing would inactivate the immobilized enzyme. Low temperatures slow-down denaturation processes and microbial contamination. The lipase TLL immobilized on rice husk retains its initial activity for at least 8 months when stored at 5 °C.

The storage can be a liquid phase storage or a dry storage depending on the optimal storing conditions for the native agents, to be considered as available in prior art as a general knowledge for expert in the field. For example, Glucoamylase needs to be stored in water, otherwise literature shows that it loses its activity when stored in dry conditions. In the case of lipases (TLL, CaLB) dry storage is possible and recommended because they have to be used in non-aqueous media and they retain their activity even when poorly hydrated.

The rice husk particles with immobilized agents, as for example enzymes, brings one further remarkable advantage: the presence of silica confers stability towards microbial or molds attack. As a consequence, the rice husk particles with immobilized agents according to the method of the present invention can be stored even in a solid form containing a small amount of hydration water (5-14%) without observing the growth of molds on the surface. On the contrary, when enzymes are immobilized on most common lignocellulosic biomass (e.g. cellulose, crosslinked polysaccharides) the water retained by biopolymers that creates a favorable ground for molds growth. We observed the growth of molds after 4 weeks on enzymes immobilized on cellulosic biomass different from rice husk, not containing silica. Concerning the rice husk particles with immobilized agents according to the method of the present invention, it must be underlined that the final product deriving from the disclosed method is a new product because:
- no existing commercial formulation of carriers with firmly immobilized agents are available which have the characteristics of the rice husk particles with firmly immobilized agents resulting from the method according to the present invention;
- no existing commercial formulation of carriers with firmly immobilized agents are available showing the mechanical and chemical stability of the rice husk particles with firmly immobilized agents resulting from the method according to the present invention.

The method according to the present invention provides advantageously a final product, that is carriers with firmly immobilized agents in the form of rice husk particles with firmly immobilized agents, which has a natural origin, has a low price, provides a large surface for the interaction of the immobilized agents in subsequent processes, has very low density.

Applying the method of the invention, a binding of at least one immobilized agent on the rice husk particles is obtained which:
- is stronger than prior art methods which do not make use of chemicals (e.g. additional crosslinking agents such as glutaraldehyde) or of supports produced/modified through a specific chemical procedure aimed at the introduction of functional groups necessary to establish covalent bonds between the agent and the support;
- equivalent or at least strong enough for successive use in reactions with respect to prior art methods which do provide the use of chemicals (binders, crosslinking agents, such as glutaraldehyde) or of supports produced/modified through a specific chemical procedure aimed at the introduction of functional groups necessary to establish covalent bonds between the agent and the support).

The method of the invention is based on a process which is intended to have as a result an alternative immobilization mechanism which is obtained due to the penetration of the agents inside cavities, tunnels and pores of the carrier material, that is rice husk particles having the size as previously disclosed, establishing strong interactions between the lignocellulosic surface and the silica of the matrix of the carrier material and the enzyme or protein. Despite the method of the invention is based on a process which is intended to have as a result an alternative immobilization mechanism with respect to known processes based on covalent bonding, some covalent bond can take place between the agents, for example enzymes, and the rice husk particles, for instance with the silica content, and other different factors can contribute to the firm immobilization, as ionic or Van Der Waals forces. However, when applying the method of the invention, covalent bonding or other immobilization mechanisms are a residual minority with respect to the described obtained effect of penetration of the agent/agents into the cavities, tunnels and pores of the carrier material establishing strong interactions between the lignocellulosic surface and the silica forming the matrix of the rice husk particles and the agent.

### EXAMPLES THAT DEMONSTRATE THE FIRM IMMOBILIZATION OF THE ONE OR MORE AGENTS AND COMPARISON WITH PRIOR ART

Immobilization of glucoamylase: The demonstration that the enzyme glucoamylase is firmly immobilized is reported in EXAMPLE 3: the column made of glucoamylase immobilized on rice husk (previously rinsed as described in EXAMPLE 3) was maintained in operation for 23 days. During each day, the free active enzyme which is present in the column effluent (i.e. detached from the column) was assessed by measuring the hydrolytic activity of the solution. The leaching of the enzyme from the solid support was determined by analyzing the glucose concentration in the same sample twice: at sample collection, and after incubating the sample at 25°C for 24 hours, and then subtracting the initial value from the final value. The resulting value are plotted in Fig. 7 representing the results of the leaching evaluation experiments in aqueous systems carried out in continuous flow column (measured at both 20 micro liters/min and 30 micro liters/min flow). In total, the column was maintained in operation for 23 days, and no significant leaching was observed (the amount of active detached protein is within the experimental error).

Moreover, the stability of the immobilized enzyme is demonstrated by the stable average activity under 23 days operation under continuous flow system and by the stable activity of glucoamylase immobilized on rice husk employed in aqueous solution and batch system (EXAMPLE 4b and Fig. 16) demonstrating that the activity of the immobilized enzyme is constant over the course of four activity text cycles (each cycle lasting 3 hours).

### COMPARISON WITH PRIOR GLUCOAMYLASE IMMOBILIZATION METHODS

Methods are known for glucoamylase covalent immobilization on commercially available methacrylic resin functionalized with amino groups and activation with glutaraldehyde or 2,5-diformylfuran (DFF). A reaction with prior glucoamylase immobilization methods was monitored for 14 days under the same experimental conditions in a continuous flow activity assay, in which 150 mg of wet weight immobilized enzyme were introduced in a 10 mL glass column. The column was filled with a 25% maltose solution in 10 mM citrate buffer, pH 4.5. A continuous flow of maltose solution was supplied to the column at a rate of 0.15 mL/min over the course of 13 days. Every day, a sample (3-4 mL) of effluent was collected. Of this sample, 50 micro-liters were immediately diluted 10 times with HCl 0.1 M, to be used for glucose concentration analysis; the rest was used for glucoamylase leaching evaluation. At the end of the experiment, the effluent samples were analyzed to determine enzyme leaching. The enzyme leaching, evaluated by measuring the hydrolytic activity of the solution, resulted to be significant during the initial four days of operation. The leaching was confirmed by a loss of enzymatic activity during the initial four days of 25% and 30% respectively. Notably, the stability and absence of leaching for the glucoamylase immobilized in rice husk according to the present invention was studied for 23 days at 25° C in continuous flow system (see EXAMPLE 4), evidencing a remarkable effect of the method according to the present invention for achieving a firm immobilization of the agents on rice husks particles.

Methods are known for glucoamylase covalent immobilization on amino functionalized siliceous mesocellular foam, preactivated with glutaraldehyde. The foam was synthetized by using triblock co-polymer surfactant Pluronic P123 (poly(ethylene oxide)-block-poly (propylene oxide)-block-poly (ethylene oxide), with TMB (Trimethyl benzene) as organic swelling agent, dissolved in 2 M HCl containing TMB and mixed with silica precursor TEOS. The synthesis was carried out by treating the precursor gel at 100 C° for 24 h in Teflon lined autoclave. The synthesized foam material was amino functionalized by condensing 0.5 g solid with 5 mmol 3-APTES solution. Products were cooled separated by filtration washed with soxhlet extraction and dried at 60°C for 6 h. After glucoamylase immobilization, the biocatalyst was washed with aqueous buffer solution until no protein was detected in the aqueous phase. Even if low level of leaching of enzyme were attained with these supports, it will be apparent to expert in the sector that this prior art method is complicated if compared to the method according to the present invention and, moreover, this prior art method is polluting and expensive, with poorer performance as compared with the method according to the present invention.

### EXAMPLES THAT DEMONSTRATE THE FIRM IMMOBILIZATION OF LIPASES TLL AND CALB AND COMPARISON WITH PRIOR ART

Lipases are mainly applied in industry for fats and oils transformation, that means that they operate prevalently in low water hydrophobic media or in aqueous emulsion. When used in aqueous emulsion using prior art immobilization methods, the enzymatic molecules tend to detach from the support and dissolve in the aqueous phase, unless covalent bonds are established between the protein and the support, whereas in hydrophobic environment, the immobilized enzymes have a lower tendency to partition into the reaction medium. Instead, considering the method according to the present invention, the firm immobilization of lipase TLL when immobilized on rice husk according to EXAMPLE 5A (400 mg scale of immobilization) and EXAMPLE 5B (120g scale of immobilization) is demonstrated by the absence of free active enzyme in the product of the interesterification as for Fig. 17, showing Lipase TLL released during 8 interesterification cycles, assessed by measuring the hydrolytic activity present in the raw reaction product obtained from the interesterification reaction (EXAMPLE 9) of triolein and tristearin using TLL immobilized as described in EXAMPLE 5B. For the measurement of the hydrolytic activity in the reaction mixture, a sample of 50 microliters of product was withdrawn at the end of each interesterification cycle (24h each) and this sample was tested in aqueous emulsion to detect hydrolytic activity towards tributyrin as described in the hydrolytic activity evaluation procedure . The absence of detectable hydrolytic activity in all the product mixtures assessed, indicate that the active enzyme does not detach from the support over 8 cycles at 70°C, corresponding to a total of 192 h of operational use of the immobilized biocatalyst. Advantageously, rice husk particles with immobilized agents according to the present invention maintain its original morphology after 192 h of operational application (8 cycles of 24 h) at 70°C (EXAMPLE 9), as shown in Fig. 18A and Fig. 18B, representing microscope images of the TLL immobilized on rice husk respectively before and after the interesterification process.

### COMPARISON WITH OTHER TECHNOLOGIES PREVIOUSLY USED FOR LIPASE IMMOBILIZATION

A known method in prior art provides a commercial formulation of Lipozyme TL-IM used in interesterification (low water media), for example on the interesterification of palm olein using Lipozyme TL-IM, which is formulated as aggregates of enzyme and water-soluble binders. After 100 h of interesterification process the decrease of the protein content in the enzymatic formulation is negligible (close to the experimental error) and the immobilized TL IM retains 27.5 % of its activity. Notably, using the solution according to the present invention, TLL immobilized on rice husk according EXAMPLE 7B retains 27% of activity after 192 h of operational application (8 cycles of 24 h at 70°C), which evidences an improvement with respect to this prior art solution but with no need of binders which is an additional advantage over prior art methods, allowing also the recycling of rice husks at the end of the use cycles since no additional binders are used and they are easily biodegradable in soil or recyclable for different uses. In conclusion, prior art methods are known in which enzymatic protein does not detach from the commercial formulation when exposed to hydrophobic media. However the method according to the present invention applied to TLL immobilized on rice husks evidences that properties are maintained even after tests carried out for a longer time and they are stable under harsh conditions.

### COMPARISON WITH THE METHOD DISCLOSED IN US 5,776,741

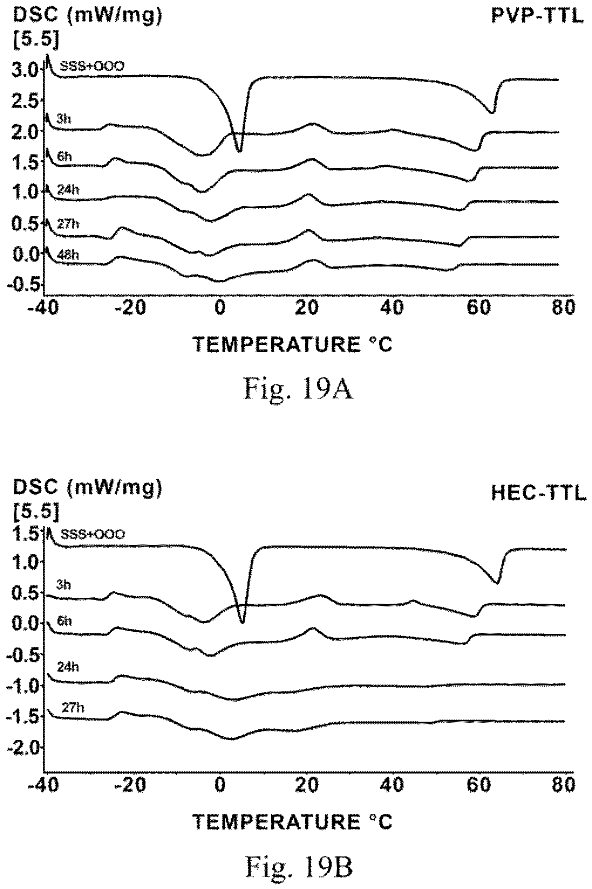

This comparison relates to use of TLL immobilized on rice husk in a fluidized reactor in the presence of water-soluble binders as PVP (polyvinylpirrolidone) and HEC (hydroxyethyl cellulose) and employed in interesterification (low-water media). TLL was immobilized on rice husk particles by mimicking the protocol reported in patent US 5,776,741 and applied in the interesterification as described in EXAMPLE 5. Fig. 19A and Fig. 19B illustrate DSC thermograms of the raw products of the interesterification between tristearin and triolein (see EXAMPLE 7) performed using immobilized lipase TLL, where the enzyme was attached on the rice husk fragments in a fluidized bed reactor employing a diluted proteic solution and water soluble binders. Reactions were performed using 560 U per gram of substrate in the case of PVP-TLL and 348 U per gram of substrate in the case of HEC-TLL. Unlike the TLL immobilized according EXAMPLE 5A and EXAMPLE 5B (Fig. 9), the immobilized TLL-PVP (Fig. 19A) and TLL-HEC (Fig. 19B), decreased significantly their efficiency in catalyzing the interesterification of triolein with tristearin, as evidenced by the slowing down of the conversion of the tristearin signal (melting peak) after 24h, particularly evident in the case of TLL-PVP (Fig. 19A). In conclusion, the formulation obtained in the presence of water soluble binders is less stable if compared with the method according the present invention.

### DEMONSTRATION OF FIRM IMMOBILIZATION OF LIPASES IMMOBILIZED ON RICE HUSK WHEN USED IN AQUEOUS EMULSION AND COMPARISON WITH PRIOR METHODS MAKING USE OF COMMERCIAL FUNCTIONALYZED RESINS ABLE TO ESTABLISH COVALENT BONDS WITH THE AGENTS

In prior art documentation relative to the use of lipases immobilized on a carrier, few or no data at all are reported relatively to the analysis of the possible detachment of lipases from the support when incubated in aqueous emulsions. In most cases residual enzymatic particles not firmly attached on the support are present because one the objective is to sell highly active immobilized enzymes, and the aim is achieved by overloading the support with an excess of enzyme. Therefore, the applicant of the present patent application collected data autonomously, by immobilizing the same amount of protein on rice husk and on the commercial methacrylic resin EC-EP (epoxy functionalized), able to establish covalent bonds with the protein. Results indicate that there is no significant release of lipases from the lipases immobilized on rice husk nor on EC-EP. However, lipase TLL immobilized on rice husk display a much higher activity. The firm immobilization of lipases TLL and CALB on rice husk (immobilized according to EXAMPLES 5A, 5B and 6) is confirmed by data in Fig. 20, which reports the measurements of the active enzyme released upon prolonged incubation (14 min for subsequent cycles) in an aqueous emulsion of the lipases immobilized on rice husk. The emulsion is more effective than water alone in removing protein particle not firmly attached on the support (Fig. 20). As a comparison, the same amounts of TLL (2mg per g of support) and CALB (14.9 mg per g of support) were also immobilized on a commercially available methacrylic resin functionalized with epoxy groups (Relizyme EC-EP, which are able to establish covalent bonds with the enzymes. In this case the enzyme was immobilized according to the conventional protocols known in the sector and based on the incubation of the carrier in a diluted aqueous solution of the enzyme and subsequent washing with an aqueous solution. Data indicate that the amount of active enzyme (i.e. hydrolytic activity detectable in 500 microliters of emulsion) detached from EC-EP (Fig. 20, column 1) or rice husk (Fig. 20, column 2, prepared according to example 5B) and subjected to the washing step before the final drying step is not detectable or negligible (within the range of experimental error). TLL immobilized on rice husk and washed shows the same behavior also after 1 month of storage (Fig. 20, column 3). In the case of TLL immobilized on rice husk but not washed prior to the final drying step and storage (Fig. 20, column 5) data indicate that some detachment of TLL in the aqueous emulsion solution occurs during the first cycle of the hydrolysis whereas the leaching during the following cycles is negligible. Therefore, lipase TLL immobilized on rice husk according EXAMPLE 5A and EXAMPLE 5B and subjected to the washing step before the final drying are firmly bound on the solid support. A minor removal of enzyme particles occurs during the washing step or, as alternative, such amount is removed during the first step of the hydrolytic reaction. On the contrary, when TLL was immobilized on rice husk by incubating the solid particles in an aqueous solution (1g of rice husk in 10 mL of 3M NaCl, for 24h) and crosslinked (addition of 100 mg of DFF in 2.5 mL of hexane, filtering and drying for 24 h) the formulation was active (Fig. 20, column 5) but the enzyme detached from the support for 5 cycles of exposure to the aqueous emulsion (14 min each). These data indicate that the establishment of firm interaction between the enzyme and the rice husk occur only when a concentrated enzymatic solution is spread directly on the carrier, thus forcing the enzyme to enter into close contact with the rice husk surface and enter rice husk cavities, tunnels and pores. As a matter of fact, the formulation of Fig. 20, column 5 was also dried for 24 h but that procedure was insufficient for establishing firm interaction between the rice husk and the lipase. Notably, the same amount of lipase TLL immobilized on rice husk and washed before storage leads to a formulation one order of magnitude more active than the preparation obtained by using the commercial epoxy acrylic resin. Furthermore, the TLL immobilized on rice husk which was not washed with water before storage leads to a formulation with a two folds higher hydrolytic activity as compared to the same TLL preparation washed before storage. This behavior is not caused by the removal of active enzyme during the washing step but rather caused by the exposure of the enzyme to a diluted aqueous phase that induces an unfavorable close conformation. It must be noted that TLL immobilized on RH has an initial activity about from 6 to 17 folds higher that TLL immobilized on EC-EP. The most unfavorable situation occurs during the immobilization on EC-EP since the enzyme undergoes a prolonged incubation in aqueous buffer, namely an environment that promotes the closing of the active site of the enzyme and is followed by the establishment of covalent bonds that rigidify and maintain the enzyme in such conformation. Therefore, the novel immobilization methodology described in EXAMPLE 5A and EXAMPLE 5B, which does not make use of a diluted aqueous solution of the enzyme, allows to prevent the unfavorable closing of the active site of those lipases that undergo interfacial activation. Moreover, rice husk, being a composite material with a hydrophobic lignin surface and a more hydrophilic cellulosic core, offers an optimal environment for the acquisition of an active-open conformation. A fast-washing step of the TLL immobilized on rice husk cause a partial conformational modification of the lipases towards a close conformation, which, however is reversible upon exposure of the biocatalyst to the hydrophobic substrates (e.g. triolein, tristearin, tributyrin). In the case of the immobilization of TLL on EC-EP the close conformation of the enzyme is favored and the formation of covalent bonds prevent further conformational changes necessary to open the active site. This effect is confirmed by the different behavior (Fig. 20, columns 7, 8, 9) of the immobilized lipase CALB, which displays a comparable activity when immobilized on RH and the epoxy acrylic resin. As a matter of fact, it is known that CALB lipase does not undergo significant conformational modification (i.e. interfacial activation leading to the opening of the active site) but rather its active site is always accessible. Therefore, the detrimental effect of the covalent immobilization on EC-EP is not evident in the case of lipase CALB.

### COMPARISON OF FIRM IMMOBILIZATION OF TLL ON RICE HUSK WITH COMMERCIAL COVALENTLY IMMOBILIZED TLL

Covalently immobilized, Lipase 76546 is commercialized by Sigma^{®} and is made by lipase TLL immobilized on the polyacrylic epoxy carrier Immobead 150. This commercial covalently immobilized TLL when used in 3 cycles of hydrolysis of tributyrin, which implies the exposure to an aqueous emulsion (14 min each cycle), releases 1.2 U in 500 microliters of emulsion upon the first cycle, 1.3 U after the second cycle and 1.3 U after the third cycle. Moreover, the resin undergoes a visible erosion with the release of fine solid particulates in the reaction medium. Therefore, despite the use of the epoxy groups on the surface of the carrier, Lipase 76546 is less firmly immobilized than TLL immobilized on rice husk according to EXAMPLES 5 and 5B. In conclusion, TLL and CALB immobilized on rice husk according to EXAMPLES 4, 5, 5B and 6 are firmly immobilized on the rice husk carrier, at least as firmly as when they are covalently immobilized on epoxy methacrylic resin Relizyme^{™} and more firmly than TLL covalently immobilized on epoxy polyacrylic resin Immobeads.

### COMPARISON WITH PRIOR IMMOBILIZATION PROTOCOLS WHICH DO NOT MAKE USE OF METHODS FOR THE FORMATION OF COVALENT BONDS BETWEEN THE SUPPORT AND THE LIPASES

The producer of the commercial formulation Lipozyme^{®} TL-IM (Novozymes^{®}) states that such formulation disaggregates at water concentration >50%, since the protein is physically immobilized, and no covalent bond takes place. When the formulation TLL-PVP and TLL-HEC (lipase TLL adsorbed on rice husk in a fluidized ben in the presence of water-soluble binders) are incubated in aqueous emulsion the enzyme detaches from the rice husk (> 90% after 14 min of incubation).

### STABILITY OF TLL IMMOBILIZED ON RICE HUSK

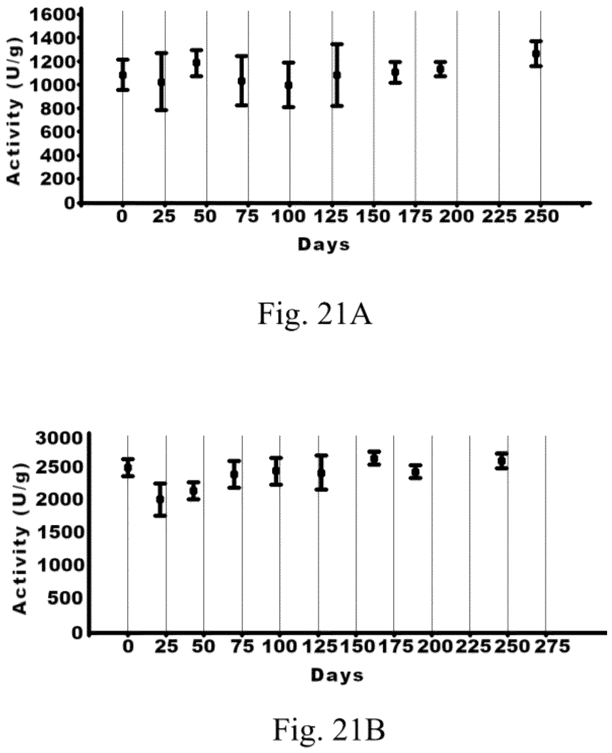

Long term stability of TLL upon 8 month storage at 5 °C was evaluated. The activity of two formulations of lipase TLL immobilized on rice husk (EXAMPLE 5B) stored at 5°C was monitored for more than 8 months. Fig. 21A illustrates the hydrolytic activity of TLL immobilized on rice husk according EXAMPLE 5B assessed for a period of 8 months washed before subsequent drying and storage. Fig. 21B illustrates the hydrolytic activity of TLL immobilized on rice husk according EXAMPLE 5B assessed for a period of 8 months without being washed before subsequent drying and storage. Fig. 21A and Fig. 21B, indicate its stability over the whole time frame considered. The washing of the immobilized TLL priori to final drying and storage did not affect the stability of the biocatalyst.

### STABILITY AND RECYCLABILITY OF TLL IN LOW-WATER MEDIA

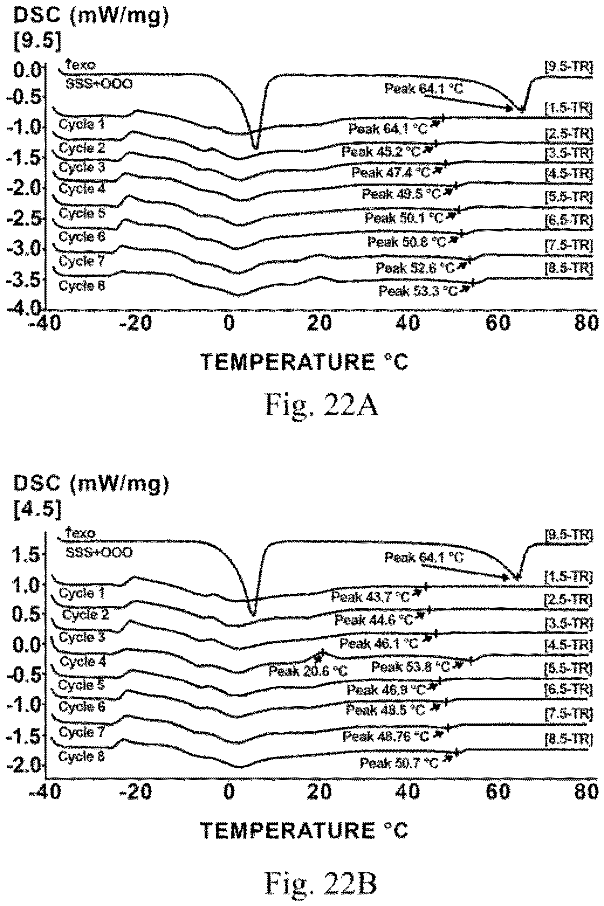

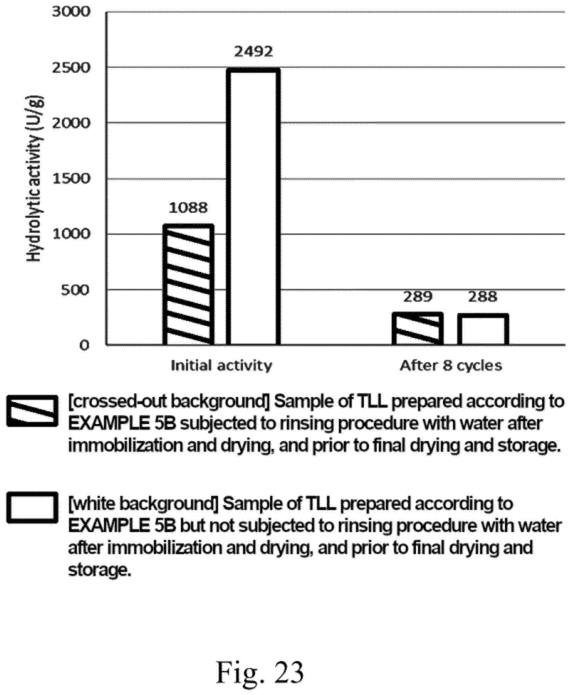

The stability of TLL immobilized on rice husk according to EXAMPLE 5A and 5B is demonstrated by the efficiency of the enzyme in catalyzing 8 cycles of interesterification at 70°C for a total of 8 days of exposure to the experimental conditions. The immobilized TLL displays comparable efficiency regardless of the washing step prior to final drying and storage as shown in Fig. 22A and Fig. 22B. Fig. 22A illustrates DSC thermograms of the raw products of the interesterification, as for example 7, for a sequence of 8 consecutive synthetic cycles relative to a process catalyzed by TLL immobilized on rice husk according to EXAMPLE 5B and washed with water after drying and prior to final drying and storage. Fig. 22B illustrates DSC thermograms of the raw products of the interesterification, as for example 7, for a sequence of 8 consecutive synthetic cycles relative to a process catalyzed by the same immobilized enzyme but stored without being washed with water. The non-washed biocatalyst (Fig. 23, columns with white background) has an initial activity two folds higher respect to the washed formulation (Fig. 23, columns with crossed-out background), but after 8 cycles of interesterification (as for example 7) the non-washed formulation (Fig. 23, columns with white background) reaches and preserves the same hydrolytic activity as the washed one (Fig. 23, columns with crossed-out background), The washed formulation retains 26.5% of its original hydrolytic activity. Data of Fig. 23 suggest that the washing step causes a temporary and reversible reduction in TLL activity, most probably as the consequence of the shifting of the conformation of the TLL towards the close non active form. However, upon exposure to the hydrophobic reaction mixture (triolein and tristearin) lipase TLL assumes its open conformation and expresses the same activity as the non-washed formulation. As a matter of fact, after 8 cycles of interesterification both formulations express a hydrolytic activity of about 290 U/g.

### STABILITY AND RECYCLABILITY OF TLL AND CALB IMMOBILIZED ON RICE HUSK IN AQUEOUS EMULSION (HYDROLYSIS OF TRIBUTYRIN)

When used in aqueous media and especially in aqueous emulsion, the enzymatic molecules tend to detach from the support and dissolve in the aqueous phase, unless covalent bonds are established between the protein and the support. Moreover, emulsions accelerate the denaturation of proteins. TLL and CALB immobilized on rice husk can be used in aqueous for the hydrolysis of tributyrin and recycled for at least 6-7 times (Fig. 24), where each cycle implies 14 min of incubation at 30 °C. Enzymes immobilized according EXAMPLE 5A, EXAMPLE 5B and EXAMPLE 6 are highly stable under mechanical mixing because the biocatalyst consists of single rice husk particles. The rice husk particles remain intact as demonstrated by microscopy images (Fig. 18A, Fig. 18B) and visual inspection of the biocatalyst exposed to aqueous emulsion (tributyrin hydrolysis assay).

In conclusion, the present invention provides a method for immobilization of one or more agents on a carrier comprising rice husks in which the method comprises at least the following steps:
(A) providing the rice husks;
(D) providing one or more agents to be immobilized on rice husks;
(F) preparation of a liquid solution in water of the one or more agents with a desired concentration (AC) of the one or more agents obtaining an agents' liquid solution;
(L) adding a quantity (QS) of the agents' liquid solution to an amount (ARH) of rice husks obtaining a mixture of agents' liquid solution and rice husks;
(M) mixing the mixture of agents' liquid solution and rice husks;
(N) drying;
and in which the rice husks are in the form of machined rice husk particles having a size in the range 100-1000 micron, preferably in the range 150-800 micron, the optimal range being 200-400 micron, and in that the rice husk particles have not being subjected to chemical modifications and the rice husk particles have not being pre-treated with crosslinking additives, the step (L) providing that the quantity (QS) of the agents' liquid solution added to the amount (ARH) of rice husks particles is such that the ratio (RL) between the quantity (QS) of the agents' liquid solution and the amount (ARH) of rice husks particles is in the range $\frac{50 micro - liters}{100 milligrams} < RL < \frac{170 micro - liters}{100 milligrams}$ so that the step (L) is a wetting phase of the rice husks particles with the agents' liquid solution with obtainment of a wet composition, the mixing step (M) being a mixing step of wetted rice husks particles promoting a homogeneous adsorption and absorption of the agents' liquid solution on the rice husk particles, in such a way that after the mixing step (M) wet rice husk particles are obtained in which water molecules of the agents' liquid solution are adsorbed and absorbed onto the rice husk particles surface and adsorbed into cavities, tunnels and pores of the rice husk particles so that no bulk water of the agents' liquid solution is present after adsorption and absorption, the one or more agents being trapped by interactions between the agents and the lignocellulosic matrix and the silica obtaining distinct fragments of rice husk particles containing the one or more agents with a loading corresponding to the concentration (AC).

With reference to adsorption and absorption:
- adsorption is the process by which a solid holds molecules of a gas or liquid or solute as a thin film;
- absorption is the process of taking something into another substance.

The interactions between the water, the agent and the rice husk are established through both mechanisms: from one side interactions are established with the wide surface of the rice husk. Secondly, the agent solution penetrates into pores and tunnels were the agent remains entrapped upon drying of the rice husk fragments, so that no bulk water of the agents' liquid solution is present after adsorption and absorption. This is crucial because in principle the adsorption of certain % of enzyme could be also obtained by incubating the rice husk in a large volume of enzymatic solution and then filtering the rice husk with the immobilized enzyme. But in the inventive method, adsorption and absorption are provided in which the whole volume of the agents' liquid solution penetrates into pores and tunnels of the rice husk particles.

When referring to rice husk particles which have not been subjected to chemical modifications and rice husk particles which have not being pre-treated with crosslinking additives, it will be understood by experts in the sector that rice husk particles do not comprise epoxide groups, binder, such as polyvinylpyrrolidone (PVP), cellulose derivatives, hexamethylenediamine (HMDA), glutaraldehyde and rice husk particles are not subjected to chemical functionalization to introduce chemical groups able to form covalent bonds with agents, as for example the in case of chemically oxidized rice husks (RH). Therefore, no new chemical functional groups through synthetic pretreatments are introduced on rice husk particles.

The distinct fragments of rice husk particles containing the one or more agents prepared according to the method of the invention are characterized by no toxicity, that is zero toxicity, in which toxicity is to be intended as the degree to which a substance can damage an organism.

The drying step (N) is preferably carried out for a drying time in the range of 12-48 hours, for letting the agents' solution penetrating into the cavities, tunnels and pores of the rice husk particles for creating interactions between the agents and the lignocellulosic matrix and the silica. For example, the drying step (N) can be carried out until a maximum quantity of possible residual water molecules is obtained which is in the range 1-20 % w/w with respect to the weight of the distinct fragments of rice husk containing the one or more agents, preferably in the range 4-15 % w/w, even more preferably in the range 7-11 % w/w.

The drying step (N) preferably comprises a first sub-step which is a spreading step in which the distinct fragments of rice husk containing the one or more agents obtained after the mixing step (M) are spread on a drying surface and a second sub-step which is a drying phase occurring at a temperature comprised between 5 and 50 °C, at environmental pressure, for a drying time in the range of 12-48 hours, for letting the agents' solution penetrating into the cavities, tunnels and pores of the rice husk particles for creating interactions between the agents and the lignocellulosic matrix and the silica. The spreading step provides spreading the distinct fragments of rice husk containing the one or more agents on the drying surface with a spreading thickness corresponding to the thickness of a single layer of rice husk particles. Preferably the concentration (AC) of the one or more agents in the agents' liquid solution is such that after the drying step (N), the loading of one or more agents on rice husks particles is obtained in which the agent's content is in the range of 0.4-150 mg per gram of rice husks particles, preferably in the range of 1-100 mg per gram of rice husks particles.

The one or more agents are firmly immobilized on the distinct fragments of rice husk particles, with the term "firm immobilization" being intended that the distinct fragments of rice husk particles containing the one or more agents are such that agents' detachment from distinct fragments of rice husk particles is lower than 3%, preferably 0%, based on the initial loading of the agents corresponding to the concentration (AC), after the distinct fragments of rice husk particles containing the one or more agents are subjected to 8 consecutive cycles of use for reactions catalyzed by the immobilized agents, as assessed by measuring the presence of the one or more agents in the raw reaction product obtained from the reactions catalyzed by the immobilized agents.

With the term "firm immobilization" referred to the agents immobilized on rice husk particles in the form of distinct fragments of rice husk particles containing the one or more agents, it is intended that the distinct fragments of rice husk particles containing the one or more agents loaded according to the disclosed method, as for example disclosed in examples 1, 2, 5A, 6, are such that agents' detachment from distinct fragments of rice husk particles is lower than 3%, preferably 0%, based on the initial loading of the agents corresponding to the concentration (AC), when the distinct fragments of rice husk particles containing the one or more agents are subjected to 8 consecutive cycles of use for reactions catalyzed by the immobilized agents, as assessed by measuring the presence of the one or more agents in the raw reaction product obtained from the reactions catalyzed by the immobilized agents. For example, in the case of an interesterification reaction (EXAMPLE 9) of triolein and tristearin using TLL immobilized as described in EXAMPLE 5B, the detection whether the interesterification product has been contaminated by free enzymatic particles that detach from the rice husk during the process can be performed by measuring the hydrolytic activity in the reaction mixture withdrawn at the end of the eighth interesterification cycle as described in the HYDROLYTIC ACTIVITY EVALUATION PROCEDURE. The absence of detectable hydrolytic activity in all the product mixtures assessed (Fig. 17), indicate that the active enzyme does not detach from the support over 8 cycles at 70°C, corresponding to a total of 192 h of operational use of the immobilized biocatalyst.

The one or more agents are preferably selected between enzymes comprising natural enzymes extracted or present in living organism and enzymes obtained by genetic engineering and manipulation, enzymes belonging to any class of the six classes defined by the International Union of Biochemistry (IUBMB): EC 1 Oxidoreductases, EC 2 Transferases, EC 3 Hydrolases, EC 4 Lyases, EC 5 Isomerases, EC 6 Ligases / synthetases, protein or biological macromolecule able to catalyze a chemical reaction, bio-stimulants for agriculture, agent for bioaugmentation for soil for use in agriculture, nutrients, ammonia, phosphate, biofertilizers and biopesticides for the soil or plants for use in agriculture, microbiota for soil improvement.

The present invention provides also the use of an agent preparation comprising a carrier and one or more catalyst agents immobilized on the carrier for reactions catalyzed by the immobilized agents, in which the agent preparation comprises distinct fragments of rice husk particles containing the one or more catalyst agents immobilized on the distinct fragments of rice husk particles according to the disclosed method for immobilization.

The present invention also relates to rice husk containing one or more agents immobilized on rice husks in which the rice husks are in the form of distinct fragments of rice husk containing the one or more agents in which the distinct fragments of rice husk have a size in the range 100-1000 micron and in that the distinct fragments of rice husk are free from chemical or crosslinking additives, in which the a loading of one or more agents on the distinct fragments of rice husk is present with an agent's content in the range of 0.4-150 mg per gram of distinct fragments of rice husk, preferably in the range of 1-100 mg per gram of distinct fragments of rice husk, in which water molecules of an agents' liquid solution are in an adsorbed and absorbed condition by the distinct fragments of rice husk surface and into cavities, tunnels and pores of the distinct fragments of rice husk, the one or more agents being trapped by interactions between the agents and the lignocellulosic matrix and the silica.

The description of the present invention has been made with reference to the enclosed figures in a preferred embodiment, but it is evident that many possible changes, modifications and variations will be immediately clear to those skilled in the art in the light of the previous description. Thus, it must be underlined that the invention is not limited to the previous description, but it includes all the changes, modifications and variations in accordance with the appended claims.

## Claims

1. Method for immobilization of one or more agents on a carrier comprising rice husks in which the method comprises at least the following steps:
(A) providing the rice husks;
(D) providing one or more agents to be immobilized on rice husks;
(F) preparation of a liquid solution in water of the one or more agents with a desired concentration (AC) of the one or more agents obtaining an agents' liquid solution;
(L) adding a quantity (QS) of the agents' liquid solution to an amount (ARH) of rice husks obtaining a mixture of agents' liquid solution and rice husks;
(M) mixing the mixture of agents' liquid solution and rice husks;
(N) drying;
**characterized in that**
the rice husks are in the form of machined rice husk particles having a size in the range 100-1000 micron and **in that** the rice husk particles have not being subjected to chemical modifications or pre-treated with crosslinking additives, the step (L) providing that the quantity (QS) of the agents' liquid solution added to the amount (ARH) of rice husks particles is such that the ratio (RL) between the quantity (QS) of the agents' liquid solution and the amount (ARH) of rice husks particles is in the range $\frac{50 micro - liters}{100 milligrams} < RL < \frac{170 micro - liters}{100 milligrams}$ so that the step (L) is a wetting phase of the rice husks particles with the agents' liquid solution with obtainment of a wet composition, the mixing step (M) being a mixing step of wetted rice husks particles promoting a homogeneous adsorption and absorption of the agents' liquid solution on the rice husk particles, in such a way that after the mixing step (M) wet rice husk particles are obtained in which water molecules of the agents' liquid solution are adsorbed and absorbed onto the rice husk particles surface and adsorbed into cavities, tunnels and pores of the rice husk particles so that no bulk water of the agents' liquid solution is present after adsorption and absorption, the one or more agents being trapped by interactions between the agents and the lignocellulosic matrix and the silica obtaining distinct fragments of rice husk particles containing the one or more agents with a loading corresponding to the concentration (AC).

2. Method for immobilization of one or more agents on a carrier comprising rice husks according to the previous claim **characterized in that**
the drying step (N) is carried out for a drying time in the range of 12-48 hours, for letting the agents' solution penetrating into the cavities, tunnels and pores of the rice husk particles for creating interactions between the agents and the lignocellulosic matrix and the silica.

3. Method for immobilization of one or more agents on a carrier comprising rice husks according to any of the previous claims **characterized in that**
the drying step (N) is carried out until a maximum quantity of possible residual water molecules is obtained which is in the range 1-20 % w/w with respect to the weight of the distinct fragments of rice husk containing the one or more agents, preferably in the range 4-15 % w/w, even more preferably in the range 7-11 % w/w.

4. Method for immobilization of one or more agents on a carrier comprising rice husks according to any of the previous claims **characterized in that**
the drying step (N) comprises a first sub-step which is a spreading step in which the distinct fragments of rice husk containing the one or more agents obtained after the mixing step (M) are spread on a drying surface and a second sub-step which is a drying phase occurring at a temperature comprised between 5 and 50 °C, at environmental pressure, for letting the agents' solution penetrating into the cavities, tunnels and pores of the rice husk particles for creating interactions between the agents and the lignocellulosic matrix and the silica.

5. Method for immobilization of one or more agents on a carrier comprising rice husks according to the previous claim **characterized in that**
the spreading step provides the spreading of the distinct fragments of rice husk containing the one or more agents on the drying surface with a spreading thickness corresponding to the thickness of a single layer of rice husk particles.

6. Method for immobilization of one or more agents on a carrier comprising rice husks according to any of the previous claims 4 to 5 **characterized in that**
the drying surface is a chemically inert surface made of a material selected between glass, ceramic, metal.

7. Method for immobilization of one or more agents on a carrier comprising rice husks according to any of the previous claims 4 to 6 **characterized in that**
the drying surface is a net shaped surface or a surface provided with holes to favor the drying of the distinct fragments of rice husk containing the one or more agents.

8. Method for immobilization of one or more agents on a carrier comprising rice husks according to any of the previous claims **characterized in that**
the concentration (AC) of the one or more agents in the agents' liquid solution is such that after the drying step (N), the loading of one or more agents on rice husks particles is obtained in which the agent's content is in the range of 0.4-150 mg per gram of rice husks particles, preferably in the range of 1-100 mg per gram of rice husks particles.

9. Method for immobilization of one or more agents on a carrier comprising rice husks according to any of the previous claims **characterized in that**
the mixing step (M) is a mixing phase occurring for a time between 3 and 10 minutes, preferably between 4 and 6 minutes.

10. Method for immobilization of one or more agents on a carrier comprising rice husks according to any of the previous claims **characterized in that**
the one or more agents are firmly immobilized on the distinct fragments of rice husk particles, with the term "firm immobilization" being intended that the distinct fragments of rice husk particles containing the one or more agents are such that agents' detachment from distinct fragments of rice husk particles is lower than 3%, preferably 0%, based on the initial loading of the agents corresponding to the concentration (AC), after the distinct fragments of rice husk particles containing the one or more agents are subjected to 8 consecutive cycles of use for reactions catalyzed by the immobilized agents, as assessed by measuring the presence of the one or more agents in the raw reaction product obtained from the reactions catalyzed by the immobilized agents.

11. Method for immobilization of one or more agents on a carrier comprising rice husks according to any of the previous claims **characterized in that**
the one or more agents are selected between enzymes comprising natural enzymes extracted or present in living organism and enzymes obtained by genetic engineering and manipulation, enzymes belonging to any class of the six classes defined by the International Union of Biochemistry (IUBMB): EC 1 Oxidoreductases, EC 2 Transferases, EC 3 Hydrolases, EC 4 Lyases, EC 5 Isomerases, EC 6 Ligases / synthetases, protein or biological macromolecule able to catalyze a chemical reaction, bio-stimulants for agriculture, agent for bioaugmentation for soil for use in agriculture, nutrients, ammonia, phosphate, biofertilizers and biopesticides for the soil or plants for use in agriculture, microbiota for soil improvement.

12. Method for immobilization of one or more agents on a carrier comprising rice husks according to any of the previous claims **characterized in that**
the rice husks are in the form of rice husk particles having a size in the range 150-800 micron, the optimal range being 200-400 micron.

13. Use of an agent preparation comprising a carrier and one or more catalyst agents immobilized on the carrier for reactions catalyzed by the immobilized agents, **characterized in that**
the agent preparation comprises distinct fragments of rice husk particles containing the one or more catalyst agents immobilized on the distinct fragments of rice husk particles according to the method for immobilization of any of the previous claims 1 to 12.

14. Rice husk containing one or more agents immobilized on rice husks **characterized in that** the rice husks are in the form of distinct fragments of rice husk containing the one or more agents in which the distinct fragments of rice husk have a size in the range 100-1000 micron and **in that** the distinct fragments of rice husk are free from chemical or crosslinking additives, in which the a loading of one or more agents on the distinct fragments of rice husk is present with an agent's content in the range of 0.4-150 mg per gram of distinct fragments of rice husk, preferably in the range of 1-100 mg per gram of distinct fragments of rice husk, in which water molecules of an agents' liquid solution are in an adsorbed condition by the distinct fragments of rice husk surface into cavities, tunnels and pores of the distinct fragments of rice husk, the one or more agents being trapped by interactions between the agents and the lignocellulosic matrix and the silica so that no bulk water of the agents' liquid solution is present.

15. Rice husk containing one or more agents immobilized on rice husks according to the previous claim **characterized in that**
distinct fragments of rice husk have a size in the range 150-800 micron, the optimal range being 200-400 micron.

16. Rice husk containing one or more agents immobilized on rice husks according to any of the previous claims 14 to 15 **characterized in that**
a maximum quantity of possible residual water molecules is present which is in the range 1-20 % w/w with respect to the weight of the distinct fragments of rice husk containing the one or more agents, preferably in the range 4-15 % w/w, even more preferably in the range 7-11 % w/w.

17. Rice husk containing one or more agents immobilized on rice husks according to any of the previous claims 14 to 16 **characterized in that**
the one or more agents are firmly immobilized on the distinct fragments of rice husk particles, with the term "firm immobilization" being intended that the distinct fragments of rice husk particles containing the one or more agents are such that agents' detachment from distinct fragments of rice husk particles is lower than 3%, preferably 0%, based on the initial loading of the agents corresponding to the concentration (AC), after the distinct fragments of rice husk particles containing the one or more agents are subjected to 8 consecutive cycles of use for reactions catalyzed by the immobilized agents, as assessed by measuring the presence of the one or more agents in the raw reaction product obtained from the reactions catalyzed by the immobilized agents.

18. Rice husk containing one or more agents immobilized on rice husks according to any of the previous claims 14 to 17 **characterized in that**
the distinct fragments of rice husk containing the one or more agents are obtained by a method for immobilization of one or more agents on a carrier comprising rice husks according to any of the previous claims 1 to 12.

## Amended claims

### Amended claims in accordance with Rule 137(2) EPC.

1. Method for immobilization of one or more agents selected from enzyme or protein or biocatalyst on a carrier comprising rice husks in which the method comprises at least the following steps:
(A) providing the rice husks;
(D) providing one or more agents selected from enzyme or protein or biocatalyst to be immobilized on rice husks;
(F) preparation of a liquid solution in water of the one or more agents selected from enzyme or protein or biocatalyst with a desired concentration (AC) of the one or more agents selected from enzyme or protein or biocatalyst obtaining an agents' liquid solution;
(L) adding a quantity (QS) of the agents' liquid solution to an amount (ARH) of rice husks obtaining a mixture of agents' liquid solution and rice husks;
(M) mixing the mixture of agents' liquid solution and rice husks;
(N) drying;
in which
the rice husks are in the form of machined rice husk particles having a size in the range 100-1000 micron , **characterized in that** the rice husk particles have not been subjected to chemical modifications or pre-treated with crosslinking additives, the step (L) providing that the quantity (QS) of the agents' liquid solution added to the amount (ARH) of rice husks particles is such that the ratio (RL) between the quantity (QS) of the agents' liquid solution and the amount (ARH) of rice husks particles is in the range $\frac{50 micro - liters}{100 milligrams} < RL < \frac{170 micro - liters}{100 milligrams}$ so that the step (L) is a wetting phase of the rice husks particles with the agents' liquid solution with obtainment of a wet composition, the mixing step (M) being a mixing step of wetted rice husks particles promoting a homogeneous adsorption and absorption of the agents' liquid solution on the rice husk particles, in such a way that after the mixing step (M) wet rice husk particles are obtained in which water molecules of the agents' liquid solution are adsorbed and absorbed onto the rice husk particles surface and adsorbed into cavities, tunnels and pores of the rice husk particles so that no bulk water of the agents' liquid solution is present after adsorption and absorption, the one or more agents being trapped by interactions between the agents and the lignocellulosic matrix and the silica obtaining distinct fragments of rice husk particles containing the one or more agents with a loading corresponding to the concentration (AC), the drying step (N) being carried out for a drying time in the range of 12-48 hours, for letting the agents' solution penetrating into the cavities, tunnels and pores of the rice husk particles for creating interactions between the agents and the lignocellulosic matrix and the silica, and the drying step (N) being carried out until a maximum quantity of possible residual water molecules is obtained which is in the range 1-20 % w/w with respect to the weight of the distinct fragments of rice husk containing the one or more agents, preferably in the range 4-15 % w/w, even more preferably in the range 7-11 % w/w, the concentration (AC) of the one or more agents in the agents' liquid solution being such that after the drying step (N), the loading of one or more agents on rice husks particles is obtained in which the agent's content is in the range of 0.4-150 mg per gram of rice husks particles, preferably in the range of 1-100 mg per gram of rice husks particles.

2. Method for immobilization of one or more agents on a carrier comprising rice husks according to the previous claim **characterized in that**
the drying step (N) comprises a first sub-step which is a spreading step in which the distinct fragments of rice husk containing the one or more agents obtained after the mixing step (M) are spread on a drying surface and a second sub-step which is a drying phase occurring at a temperature comprised between 5 and 50 °C, at environmental pressure, for letting the agents' solution penetrating into the cavities, tunnels and pores of the rice husk particles for creating interactions between the agents and the lignocellulosic matrix and the silica.

3. Method for immobilization of one or more agents on a carrier comprising rice husks according to the previous claim **characterized in that**
the spreading step provides the spreading of the distinct fragments of rice husk containing the one or more agents on the drying surface with a spreading thickness corresponding to the thickness of a single layer of rice husk particles.

4. Method for immobilization of one or more agents on a carrier comprising rice husks according to any of the previous claims 2 to 3 **characterized in that**
the drying surface is a chemically inert surface made of a material selected between glass, ceramic, metal.

5. Method for immobilization of one or more agents on a carrier comprising rice husks according to any of the previous claims 2 to 4 **characterized in that**
the drying surface is a net shaped surface or a surface provided with holes to favor the drying of the distinct fragments of rice husk containing the one or more agents.

6. Method for immobilization of one or more agents on a carrier comprising rice husks according to any of the previous claims **characterized in that**
the mixing step (M) is a mixing phase occurring for a time between 3 and 10 minutes, preferably between 4 and 6 minutes.

7. Method for immobilization of one or more agents on a carrier comprising rice husks according to any of the previous claims **characterized in that**
the one or more agents are firmly immobilized on the distinct fragments of rice husk particles, with the term "firm immobilization" being intended that the distinct fragments of rice husk particles containing the one or more agents are such that agents' detachment from distinct fragments of rice husk particles is lower than 3%, preferably 0%, based on the initial loading of the agents corresponding to the concentration (AC), after the distinct fragments of rice husk particles containing the one or more agents are subjected to 8 consecutive cycles of use for reactions catalyzed by the immobilized agents, as assessed by measuring the presence of the one or more agents in the raw reaction product obtained from the reactions catalyzed by the immobilized agents.

8. Method for immobilization of one or more agents on a carrier comprising rice husks according to any of the previous claims **characterized in that**
the one or more agents are selected between enzymes comprising natural enzymes extracted or present in living organism and enzymes obtained by genetic engineering and manipulation, enzymes belonging to any class of the six classes defined by the International Union of Biochemistry (IUBMB): EC 1 Oxidoreductases, EC 2 Transferases, EC 3 Hydrolases, EC 4 Lyases, EC 5 Isomerases, EC 6 Ligases / synthetases, protein or biological macromolecule able to catalyze a chemical reaction, bio-stimulants for agriculture, agent for bioaugmentation for soil for use in agriculture, nutrients, ammonia, phosphate, biofertilizers and biopesticides for the soil or plants for use in agriculture, microbiota for soil improvement.

9. Method for immobilization of one or more agents on a carrier comprising rice husks according to any of the previous claims **characterized in that**
the rice husks are in the form of rice husk particles having a size in the range 150-800 micron, the optimal range being 200-400 micron.

10. Use of an agent preparation as an agent preparation of an industrial process, the agent preparation comprising a carrier and one or more catalyst agents immobilized on the carrier for reactions catalyzed by the immobilized agents, **characterized in that** the agent preparation comprises distinct fragments of rice husk particles containing the one or more catalyst agents immobilized on the distinct fragments of rice husk particles according to the method for immobilization of any of the previous claims 1 to 9.

11. Rice husk containing one or more agents selected from enzyme or protein or biocatalyst immobilized on rice husks in which the rice husks are in the form of distinct fragments of rice husk containing the one or more agents selected from enzyme or protein or biocatalyst in which the distinct fragments of rice husk have a size in the range 100-1000 micron **characterized in that**
the distinct fragments of rice husk are free from chemical or crosslinking additives, in which a loading of one or more agents selected from enzyme or protein or biocatalyst on the distinct fragments of rice husk is present with an agent's content in the range of 0.4-150 mg per gram of distinct fragments of rice husk, preferably in the range of 1-100 mg per gram of distinct fragments of rice husk, in which water molecules of an agents' liquid solution are in an adsorbed condition by the distinct fragments of rice husk surface into cavities, tunnels and pores of the distinct fragments of rice husk, the one or more agents being trapped by interactions between the agents and the lignocellulosic matrix and the silica so that no bulk water of the agents' liquid solution is present, in which the agents' liquid solution is a liquid solution in water of the one or more agents with a desired concentration (AC) of the one or more agents, the agents' liquid solution being provided in a quantity (QS) of the agents' liquid solution for an amount (ARH) of rice husks the quantity (QS) of the agents' liquid solution for the amount (ARH) of rice husks particles being such that the ratio (RL) between the quantity (QS) of the agents' liquid solution and the amount (ARH) of rice husks particles is in the range $\frac{50 micro - liters}{100 milligrams} < RL < \frac{170 micro - liters}{100 milligrams}$, a maximum quantity of possible residual water molecules being present in the rice husks which is in the range 1-20 % w/w with respect to the weight of the distinct fragments of rice husk containing the one or more agents, preferably in the range 4-15 % w/w, even more preferably in the range 7-11 % w/w.

12. Rice husk containing one or more agents immobilized on rice husks according to the previous claim **characterized in that**
distinct fragments of rice husk have a size in the range 150-800 micron, the optimal range being 200-400 micron.

13. Rice husk containing one or more agents immobilized on rice husks according to any of the previous claims 11 to 12 **characterized in that**
the one or more agents are firmly immobilized on the distinct fragments of rice husk particles, with the term "firm immobilization" being intended that the distinct fragments of rice husk particles containing the one or more agents are such that agents' detachment from distinct fragments of rice husk particles is lower than 3%, preferably 0%, based on the initial loading of the agents corresponding to the concentration (AC), after the distinct fragments of rice husk particles containing the one or more agents are subjected to 8 consecutive cycles of use for reactions catalyzed by the immobilized agents, as assessed by measuring the presence of the one or more agents in the raw reaction product obtained from the reactions catalyzed by the immobilized agents.

14. Rice husk containing one or more agents immobilized on rice husks according to any of the previous claims 11 to 13 **characterized in that**
the distinct fragments of rice husk containing the one or more agents are obtained by a method for immobilization of one or more agents on a carrier comprising rice husks according to any of the previous claims 1 to 9.
